# EUROPEAN PATENT SPECIFICATION

(11) **EP 0 604 641 B1**
(45) Date of publication and mention of the grant of the patent: **20.03.2002**
(21) Application number: 93916834.0
(22) Date of filing: 29.06.1993
(51) Int. Cl.: A61K 31/785, A61K 31/196, A61P 29/00

(54) **USE OF A COMBINATION CONTAINING AN AMINE OR AMINE-RELATED DERIVATIVE OF BENZOIC ACID AND AN AMINO-POLYSACCHARIDE IN THE MANUFACTURE OF A MEDICAMENT FOR THE TREATMENT OF INFLAMMATORY DISEASES**
VERWENDUNG EINER KOMBINATION BESTEHEND AUS EINEM AMINDERIVAT ODER AMINVERWANDTEN DERIVAT DER BENZOESÄURE UND EINEM AMINO-POLYSACCHARIDE ZUR HERSTELLUNG EINES MEDIKAMENTS FUER DIE BEHANDLUNG VON ENTZÜNDLICHEN ERKRANKUNGEN
UTILATION D'UNE COMBINAISON CONTENANT UN DERIVE AMINE OU APPARENTE AUX AMINES D'ACIDE BENZOIQUE AVEC UN AMINO-POLYSACCHARIDE DANS LA FABRICATION D'UN MEDICAMENT DESTINE AU TRAITEMENT DE MALADIES INFLAMMATOIRES

(30) Priority: 30.06.1992 US 906909
(43) Date of publication of application: 06.07.1994
(73) Proprietor: SHAPIRO, Howard, K., Narberth, PA 19072 (US)
(72) Inventor: SHAPIRO, Howard, K., Narberth, PA 19072 (US)
(74) Representative: O'Connor, Donal Henry
(86) International application number: US9306167
(87) International publication number: WO9400135

(56) References cited:
- EP-A- 0 011 092
- EP-A- 0 094 599
- WO-A-91/07420
- WO-A-92/02216
- WO-A-92/14456
- DD-A- 250 465
- DD-A- 294 707
- DE-A- 2 914 005
- DE-A- 3 237 253
- US-A- 3 926 961
- US-A- 3 956 504
- US-A- 3 957 850
- US-A- 4 017 623
- US-A- 4 272 512
- US-A- 4 277 496
- US-A- 4 584 379
- US-A- 4 851 426
- TEXAS STATE J. OF MEDICINE, vol. 49, 1953 pages 666-72, ZARAFONETIS C.J.D. 'Clinical Use of Para-Aminobenzoic Acid'
- PHARMAZIE, vol. 29, 1974 pages 333-6, WALSMANN ET AL 'Synthetische Inhibitoren von Serin Proteasen'
- BIOCHEMICAL PHARMACOLOGY, vol. 29, 1980 pages 121-4, BENEDETTI ET AL 'Foot-Edema Induced by Compounds Originating from the Peroxidation of Liver Microsomal Lipids'
- PHYSICIANS' DESK REFERENCE, Edition 45, issued 1991, page 1045.
- BIOCHEMICAL PHARMACOLOGY, Volume 38, No. 8, issued 1989, LEHMANN et al., "Substrates for Arachidonic Acid Co-Oxidation with Peroxidase/Hydrogen Peroxide", pages 1209-1216.

## Description

### I. SUMMARY OF THE INVENTION

The present invention is directed to the use of compositions consisting of amine and amine-related benzoic acid derivative primary agents capable of covalently binding carbonyl substances and co-agents according to claim 1 for use of the manufacture of a medicament for the treatment of symptoms of chronic inflammatory disorders according to claim 1, said disorders featuring oxidative free radical reactions, lipid peroxidation and generation of carbonyl compounds as aspects of their etiologies.

The invention relates to the use of a composition comprising a therapeutically effective, amount of a primary agent and at least one co-agent, said primary agent comprising a water soluble, low molecular weight primary amine or amine-related derivative of benzoic acid in the molecular weight range of 100 to 1,400, for use in the treatment of symptoms of chronic inflammatory disorders according to claim 1 featuring pathology based in part on increased lipid peroxidation, wherein said primary and co-agent combination according to claim 1 produces an anti-inflammatory and analgesic effect.

WO92/14456, a document under Art. 54 (3) EPC, discloses the use of similar compounds as described in the present invention for the treatment of polyneuropathies, in particular multiple sclerosis.

In such a preferred embodiment of said use of a composition comprising a therapeutically effective amount of a primary agent and at least one co-agent for treatment of the symptomology of a chronic inflammatory disease, the chronic inflammatory disease is characterized in part by the deterioration of intracellular and extracellular structures and is characterized by the spurious pathological chemical covalent bond crosslinking of said structures, formation of pathological addition products of said structures, or chemical cleavage products of said structures, said deterioration resulting from reaction of inflammation site structures in the human with disease-induced carbonyl-containing aliphatic and aromatic hydrocarbons resulting from increased lipid peroxidation associated with chronic inflammation.

In a preferred embodiment of this aspect of the invention, the primary agent has at least one primary amine group or amine-related group thereon for reaction with lipid peroxidation product carbonyl groups to decrease the deterioration of said intracellular and extracellular structures and to decrease the spurious pathological chemical crosslinking, other derivation or cleavage of said structures by permitting a therapeutically effective amount of said primary agent to effectively compete with and covalently bind to said discase-induced carbonyl-containing lipid peroxidation products.

In a preferred embodiment, the use of the primary agent is additionally characterized in that it does not interact with normal cell metabolism of said human or does so in a non-cytotoxic manner, and the primary agent is readily absorbed by the kidney tissue of said human and excreted in the urine of said human without nephrotoxic consequences.

In a preferred embodiment, the use of a water soluble primary agent of molecular weight in the range of 100 to 1,400 is selected from the group consisting of the free acid forms, salts, benzene ring isomers, amide derivatives, carboxylic acid ester derivatives and analogous non-aromatic benzene ring derivatives of the group consisting of: 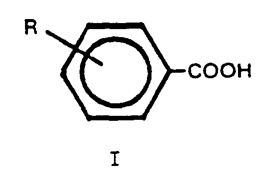
- R =: -NH₂
-aminoalkyl group having 1-10 carbons including hydrocarbon isomers and/or hydroxylated derivatives thereof
-NHC(=NH)NH₂
-(CH₂)ₙNHC(=NH)NH₂ where n = 1-10
-C(=NH)-NH₂ (continued)
-(CH₂)ₙ-CH=NC(=NH)NH₂ where n = 1-10
-NHC(=NH)NHNH₂
-(CH₂)ₙNHC(=NH)NHNH₂ where n = 1-10
- (CH₂)ₙ-CH=NC(=NH)NHNH₂ where n = 1-10
-NHNHC(=NH)NH₂
-(CH₂)ₙ-NHNHC(=NH)NH₂ where n = 1-10
- (CH₂)ₙ-CH=N-NHC(=NH)NH₂ where n = 1-10 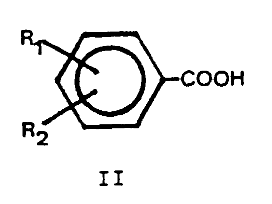
- R₁ =: -NH₂
-aminoalkyl group (1-10 carbons) including hydrocarbon isomers and/or hydroxylated derivatives thereof
-(CH₂)ₙNHC(=NH)NH₂ where n = 1-10
-C(=NH)-NH₂
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
-(CH₂)ₙ-NHNHC(=NH)NH₂ where n = 1-10
-(CH₂)ₙ-CH=N-NHC(=NH)NH₂ where n = 1-10
- R₂ =: -NH₂
-OH
-O-CH₃
-O-R' with alkyloxy group R' having 2-10 carbons including hydrocarbon isomers and/or hydroxylated derivatives thereof
-aminoalkyl group (1-10 carbons) including hydrocarbon isomers and/or hydroxylated derivatives thereof
-SO₃H
-CH₃
-(CH₂)ₙCH₃ where n = 1-10 including hydrocarbon (continued) isomers and/or hydroxylated derivatives thereof 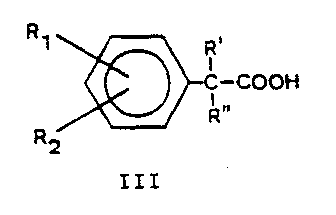
- R₁=: -(CH₂)ₙ-NH₂ where n = 0-10 including isomers of the aminoalkyl group and hydroxylated derivatives thereof
-C(=NH)-NH₂
-NHC(=NH)NH₂
-(CH₂)ₙNHC(=NH)NH₂ where n = 1-10
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
-(CH₂)ₙ-NHNHC(=NH)NH₂ where n = 1-10
-(CH₂)ₙ-CH=N-NHC(=NH)NH₂ where n = 1-10
- R₂ =: -NH₂
-H
-OH
-O-CH₃
-O-R₃ where alkyloxy group R₃ has 2-10 carbons including hydrocarbon isomers and/or hydroxylated derivatives thereof
-aminoalkyl group (1-10 carbons) including hydrocarbon isomers and/or hydroxylated derivatives thereof
-SO₃H
-CH₃
-(CH₂)ₙCH₃ where n = 1-10 including hydrocarbon isomers and/or hydroxylated derivatives thereof
- R' =: -H
-CH₃
-OH
- R" =: -H
-CH₃
-OH,
for controlling the symptoms of disorders selected from the group consisting of chronic gingivitis, chronic periodontitis, chronic autoimmunc gastritis, ileitis, colitis, interstitial cystitis, arthritis, tendinitis, carpel tunnel syndrome and other cumulative trauma disorders, systemic lupus erythematosus, autoimmune vasculitis, asbestosis, silicosis, chronic obstructive pulmonary disease, Lyme disease, inflammatory myopathies, status epilepticus, inflammatory neuropathies, myasthenia gravis, , as well as lessening of inflammatory site edema, and treatment of post-event ischemia and reperfusion symptomology resulting from acute central nervous system trauma, stroke and myocardial infarction; excluding multiple sclerosis.

In a preferred embodiment, the primary agent is used in a dosage in the range of one gram/day to 20 grams/day.

In a preferred embodiment, the primary agent is administered orally.

In a preferred embodiment, the primary agent is administered intravenously.

In a preferred embodiment, the co-agent is a non-absorbable polyamine substance or non-absorbable polyamine-related substance, a therapeutically effective amount of said co-agent acting to covalently bind to and sequester dietary carbonyl-containing products.

In a preferred embodiment, the co-agent is a non-absorbable polyamine substance or non-absorbable polyamine-related substance selected from the group consisting of:
a. naturally occurring polysaccharides having beta-1,2, beta-1,3, beta-1,4 and/or beta-1,6 linkages containing aminosugars including the chitin class of biopolymers having the general structure of poly-beta-(1→4)-N-acetyl-D-glucosamine, and bearing at least one free primary amine group;
b. deacetylated naturally occurring polysaccharides, having at least one N-acetylated residue, including chitosan, deacetylated chondroitin sulfate, or dracetylated hyaluronic acid;
c. chemically aminated polysaccharides selected from the group consisting of:
   aminodeoxy polysaccharides such as 2-amino-2-deoxycellulose;
   aminoalkyl-, amino(hydroxyalkyl)-, aminoalkyl-ether-, and
   amino(hydroxyalkyl)-ether-derivatives of cellulose, chitin and other naturally occurring non-digestible carbohydrates selected from the group consisting of
   **H**_{**2**}**N-(CH**_{**2**}**)**_{**n**}**-[carbohydrate]**, where n = 1-10, including alkyl isomers;
   **H**_{**2**}**N-(CH**_{**2**}**)**_{**m**}**-CHOH-(CH**_{**2**}**)**_{**n**}**-[carbohydrate]**, where m = 0-10 and n = 0-10;
   **H**_{**2**}**N-(CH**_{**2**}**)**_{**n**}**-O-[carbohydrate]** where n = 1-10;
   **H**_{**2**}**N-(CH**_{**2**}**)**_{**m**}**-CHOH**-**(CH**_{**2**}**)**_{**n**}**-O-[carbohydrate]** where m = 0-10 and n = 0-10;
   aminobenzyl- derivatives of cellulose, chitin or other naturally occurring non-digestible carbohydrates selected from the group consisting of
   **H**_{**2**}**N-C**_{**6**}**H**_{**4**}**-(CH**_{**2**}**)**_{**n**}**-[carbohydrate],**
   **H**_{**2**}**N-CH**_{**2**}**-C**_{**6**}**H**_{**4**}**-(CH**_{**2**}**)**_{**n**}**-[carbohydrate]**,
   **H**_{**2**}**N-C**_{**6**}**H**_{**4**}**-(CH**_{**2**}**)**_{**n**}**-O-[carbohydrate]** where n = 0 - 10, and
   **H**_{**2**}**N-C**_{**6**}**H**_{**4**}**-(CH**_{**2**}**)**_{**m**}**-CHOH-(CH**_{**2**}**)**_{**n**}**-O-[carbohydrate]** where m = 0-10 and n = 0-10, including p-, o- and m-benzene ring amino-isomers, aminomethyl- isomers and alkyl group isomers thereof;
   guanidine and aminoguanidine derivatives of cellulose, chitin or other naturally occurring non-absorbable carbohydrates selected from the group consisting of:
   **H**_{**2**}**N-C(=NH)-[carbohydrate];**
   **H**_{**2**}**N-C(=NH)-(CH**_{**2**}**)**_{**n**}**-[carbohydrate]**, where n = 1-10, including hydrocarbon isomers and hydroxylated derivatives thereof;
   **H**_{**2**}**N-C(=NH)-O-(CH**_{**2**}**)**_{**n**}**-[carbohydrate]**, where n = 1-10, including hydrocarbon isomers, ether linkage isomers and hydroxylated derivatives thereof;
   **H**_{**2**}**N-C(=NH)-NH-[carbohydrate];**
   **H**_{**2**}**N-C(=NH)-NH-(CH**_{**2**}**)**_{**n**}**-[carbohydrate],** where n = 1-10, including hydrocarbon isomers and hydroxylated derivatives thereof;
   **H**_{**2**}**N-C(=NH)-NH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-C(=NH)-N=CH-(CH**_{**2**}**)**_{**n**}**-[carbohydrate],** where n = 1-10, including hydrocarbon isomers and hydroxylated derivatives thereof;
   **H**_{**2**}**N-C(=NH)-N=CH-(CH**_{**2**}**)**_{**n**}**-O-[carbohydrate],** where n = 1-10, including hydrocarbon isomers and hydroxylated derivatives thereof;
   **H**_{**2**}**N-NHC(=NH)-NH-[carbohydrate];**
   **H**_{**2**}**N-NHC(=NH)-NH-(CH**_{**2**}**)**_{**n**}**-[carbohydrate],** where n = 1-10, including hydrocarbon isomers and hydroxylated derivatives thereof;
   **H**_{**2**}**N-NHC(=NH)-NH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-NHC(=NH)-N=CH-(CH**_{**2**}**)**_{**n**}**-[carbohydrate]**, where n = 1-10, including hydrocarbon isomers and hydroxylated derivatives thereof;
   **H**_{**2**}**N-NHC(=NH)-N=CH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-C(=NH)-NH-NH-[carbohydrate];**
   **H**_{**2**}**N-C(=NH)-NH-NH-(CH**_{**2**}**)**_{**n**}**-[carbohydrate]**, where n = 1-10, including hydrocarbon isomers and hydroxylated derivatives thereof;
   **H**_{**2**}**N-C(=NH)-NH-NH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-C(=NH)-NH-N=CH-(CH**_{**2**}**)**_{**n**}**-[carbohydrate],** where n = 1-10, including hydrocarbon isomers and hydroxylated derivatives thereof;
   **H**_{**2**}**N-C(=NH)-NH-N=CH-(CH**_{**2**}**)**_{**n**}**-O-[carbohydrate]**, where n = 1-10, including hydrocarbon isomers, ether linkage isomers and hydroxylated derivatives thereof;
d. primary amine, aminoguanidine and guanidine derivatives of sucrose polyesters having one or more carbonyl trapping functional group per molecule wherein each carbonyl trapping functional group is in the omega-, omega-1 or other isomeric position within the fatty acyl chains, wherein each fatty acyl chain may have from 3 to 26 carbons, from one to five nitrogen functional groups and from one to 24 hydroxyl groups;
e. synthetic polysaccharides consisting partly or entirely of aminosugars bound by beta-1,2, beta-1,3, beta-1,4 and/or beta-1,6 linkages;
f. mixed polysaccharide polymeric derivatives wherin primary amine, aminoalkyl (one to ten carbons per alkyl group), amino-hydroxyalkyl (one to ten carbons per alkyl group and one to ten hydroxyl groups per alkyl group), aminoguanidine, aminoguanidinylalkyl (one to ten carbons per alkyl group), aminoalkyl-guanidinyl (one to ten carbons per alkyl group), guanidine, aminobenzene and/or aminoalkylbenzene (one to ten carbons per alkyl group) functional groups are covalently attached to matrices such as cpi-chlorohydrin copolymers of cellulose or chitin and wherein hydrocarbon spacer groups may include alkene as well as alkyl groups; and
g. non-polysaccharide polymeric derivatives wherein primary amine, aminoalkyl (one to ten carbons per alkyl group), aminohydroxyalkyl (one to ten carbons per alkyl group and one to ten hydroxyl groups per alkyl group), aminoguanidine, aminoguanidinylalkyl (one to ten carbons per alkyl group), aminoalkyl-guanidinyl (one to ten carbons per alkyl group), guanidine, aminobenzene and/or aminoalkylbenzene (one to ten carbons per alkyl group) functional groups are covalently attached to a synthetic non-digestible polymer selected from the group consisting of polystyrene, styrene-divinylbenzene copolymer, polyvinyl alcohol and crosslinked derivatives thereof, and wherein hydrocarbon spacer groups may include alkene as well as alkyl groups,
for controlling the symptoms of disorders selected from the group consisting of chronic gingivitis, chronic periodontitis, chronic autoimmune gastritis, ileitis, colitis, interstitial cystitis, arthritis, tendinitis, carpel tunnel syndrome and other cumulative trauma disorders, systemic lupus crythematosus, autoimmune vasculitis, asbestosis, silicosis, chronic obstructive pulmonary disease, Lyme disease, inflammatory myopathies, status opilepticus, inflammatory neuropathies, myasthenia gravis, as well as lessening of inflammatory site edema, and treatment of post-event ischemia and reperfusion symptomology resulting from acute central nervous system trauma, stroke and myocardial infarction; excluding multiple sclerosis.

In a preferred embodiment, the co-agent is a non-absorbable polyamine substance or non-absorbable polyamine-related substance which is in a microfibrillated form or microcrystalline form having enhanced surface area, increased porosity, increased water retention capacity and enhanced chemical accessibility.

In a preferred embodiment, the therapeutically effective amount of said co-agent is a dosage in the range of one gram/day to 40 grams/day.

In a preferred embodiment, the therapeutically effective amount of said non-absorbable polyamine co-agent or non-absorbable polyamine-related co-agent is administered orally.

In another aspect of this invention, the invention relates to the use of a composition for treating a mammal suffering from a chronic inflammatory disorder featuring pathology which includes in part increased lipid peroxidation comprising orally or intravenously administering a therapeutically effective amount of a primary agent in combination with at least one co-agent, wherein said mammalian disorder is selected from a group consisting of arthritis, inflammatory site edema, acute central nervous system trauma, stroke and myocardial infarction, wherein the primary therapeutic agent is selected from the group consisting of primary amine and amine-related benzoic acid derivatives which are water soluble and of molecular weight 100 to 1,400 as defined above, wherein the primary agent acts by covalently binding to and sequestering carbonyl compounds in said mammal which result from increased lipid peroxidation, and wherein said primary agent is administered with at least one co-agent.

In a preferred embodiment, the primary agent of this invention is used to treat a mammal in a dosage in the range of 10 mg/kg/day to 1.0 gm/kg/day.

In a preferred embodiment, the non-absorbable polyamine substance or non-absorbable polyamine-related substance as defined above is used to treat a mammal in a dosage in the range of 10 mg/kg/day to 1.0 gm/kg/day.

In accordance with the present invention, the use of the pharmaceutical composition comprises the use of at least one co-agent.

In another aspect of this invention, the invention relates to a pharmaceutical composition for use in the treatment of the symptoms of disorders selected from the group consisting of:
chronic gingivitis, chronic periodontitis, chronic autoimmune gastritis, ileitis, colitis, interstitial cystitis, arthritis, tendinitis, carpel tunnel syndrome and other cumulative trauma disorders, systemic lupus erythematosus, autoimmune vasculitis, asbestosis, silicosis, chronic obstructive pulmonary disease, Lyme disease, inflammatory myopathies, status epilepticus, inflammatory neuropathies, myasthenia gravis, as well as lessening of inflammatory site edema, and treatment of post-event ischemia and reperfusion symptomology resulting from acute central nervous system trauma, stroke and myocardial infarction; excluding multiple sclerosis.
the composition comprising one or more non-absorbable polyamine co-agent or non-absorbable polyamine-related co-agent selected from the group consisting of:
   a. naturally occurring polysaccharides having beta-1,2, beta-1,3, beta-1,4 and/or beta-1,6 linkages containing aminosugars including the chitin class of biopolymers having the general structure of **poly-beta-(1→4)-N-acetyl-D-glucosamine,** and bearing at least one free primary amine group;
   b. deacetylated naturally occurring polysaccharides, having at least one N-acetylated residue, including chitosan, deacetylated chondroitin sulfate, or deacetylated hyaluronic acid;
   c. chemically aminated polysaccharides selected from the group consisting of:
      aminodeoxy polysaccharides such as 2-amino-2-deoxycellulose;
      aminoalkyl-, amino(hydroxyalkyl)-, aminoalkyl-ether-, and
      amino(hydroxyalkyl)-ether-derivatives of cellulose, chitin and other naturally occurring non-digestible carbohydrates selected from the group consisting of
      **H**_{**2**}**N-(CH**_{**2**}**)**_{**n**}**-[carbohydrate]** where n = 1-10, including alkyl isomers;
      **H**_{**2**}**N-(CH**_{**2**}**)**_{**m**}**-CHOH-(CH**_{**2**}**)**_{**n**}**-[carbohydrate]**, where m = 0-10 and n = 0-10;
      **H**_{**2**}**N-(CH**_{**2**}**)**_{**n**}**-O-[carbohydrate]** where n = 1-10;
      **H**_{**2**}**N-(CH**_{**2**}**)**_{**m**}**-CHOH-(CH**_{**2**}**)**_{**n**}**-O-[carbohydrate]** where m = 0-10 and n = 0-10;
      aminobenzyl- derivatives of cellulose, chitin or other naturally occurring non-digestible carbohydrates selected from the group consisting of
      **H**_{**2**}**N-C**_{**6**}**H**_{**4**}**-(CH**_{**2**}**)**_{**n**}**-[carbohydrate]**,
      **H**_{**2**}**N-CH**_{**2**}**-C**_{**6**}**H**_{**4**}**-(CH**_{**2**}**)**_{**n**}**-[carbohydrate]**
      **H**_{**2**}**N-C**_{**6**}**H**_{**4**}**-(CH**_{**2**}**)**_{**n**}**-O-[carbohydrate]** where n = 0 - 10, and
      **H**_{**2**}**N-C**_{**6**}**H**_{**4**}**-(CH**_{**2**}**)**_{**m**}**-CHOH-(CH**_{**2**}**)**_{**n**}**-O-[carbohydrate]** where m = 0-10 and n = 0-10, including p-, o- and m-benzene ring amino-isomers, aminomethyl- isomers and alkyl group isomers thereof;
   guanidine and aminoguanidine derivatives of cellulose, chitin or other naturally occurring non-absorbable carbohydrates selected from the group consisting of:
      **H**_{**2**}**N-C(=NH)-[carbohydrate] ;**
      **H**_{**2**}**N-C(=NH)-(CH**_{**2**}**)**_{**n**}**-[carbohydrate**], where n = 1-10, including hydrocarbon isomers and hydroxylated derivatives thereof;
      **H**_{**2**}**N-C(=NH)-O-(CH**_{**2**}**)**_{**n**}**-[carbohydrate]**, where n = 1-10, including hydrocarbon isomers, ether linkage isomers and hydroxylated derivatives thereof;
      **H**_{**2**}**N-C(=NH)-NH-[carbohydrate];**
      **H**_{**2**}**N-C(=NH)-NH-(CH**_{**2**}**)**_{**n**}**-[carbohydrate]**, where n = 1-10, including hydrocarbon isomers and hydroxylated derivatives thereof;
      **H**_{**2**}**N-C(=NH)-NH-(CH**_{**2**}**)**_{**n**}**-O-[carbohydrate]**, where n = 1-10, including hydrocarbon isomers, ether linkage isomers and hydroxylated derivatives thereof;
      **H**_{**2**}**N-C(=NH)-N=CH-(CH**_{**2**}**)**_{**n**}**-[carbohydrate]**, where n = 1-10, in-cluding hydrocarbon isomers and hydroxylated derivatives thereof;
      **H**_{**2**}**N-C(=NH)-N=CH-(CH**_{**2**}**)**_{**n**}**-O-[carbohydrate]**, where n = 1-10, including hydrocarbon isomers and hydroxylated derivatives thereof;
      **H**_{**2**}**N-NHC(=NH)-NH-[carbohydrate];**
      **H**_{**2**}**N-NHC(=NH)-NH-(CH**_{**2**}**)**_{**n**}**-[carbohydrate]**, where n = 1-10, including hydrocarbon isomers and hydroxylated derivatives thereof;
      **H**_{**2**}**N-NHC(=NH)-NH-(CH**_{**2**}**)**_{**n**}**-O-[carbohydrate]**, where n = 1-10, including hydrocarbon isomers, ether linkage isomers and hydroxylated derivatives thereof;
      **H**_{**2**}**N-NHC(=NH)-N=CH-(CH**_{**2**}**)**_{**n**}**-[carbohydrate]**, where n = 1-10, including hydrocarbon isomers and hydroxylated derivatives thereof;
      **H**_{**2**}**N-NHC(=NH)-N=CH-(CH**_{**2**}**)**_{**n**}**-O-[carbohydrate]**, where n = 1-10, including hydrocarbon isomers, ether linkage isomers and hydroxylated derivatives thereof;
      **H**_{**2**}**N-C(=NH)-NH-NH-[carbohydrate];**
      **H**_{**2**}**N-C(=NH)-NH-NH-(CH**_{**2**}**)**_{**n**}**-[carbohydrate],** where n = 1-10, ineluding hydrocarbon isomers and hydroxylated derivatives thereof;
      **H**_{**2**}**N-C(=NH)-NH-NH-(CH**_{**2**}**)**_{**n**}**-O-[carbohydrate]**, where n = 1-10, including hydrocarbon isomers, ether linkage isomers and hydroxylated derivatives thereof;
      **H**_{**2**}**N-C(=NH)-NH-N=CH-(CH**_{**2**}**)**_{**n**}**-[carbohydrate]**, where n = 1-10, including hydrocarbon isomers and hydroxylated derivatives thereof;
      **H**_{**2**}**N-C(=NH)-NH-N=CH-(CH**_{**2**}**)**_{**n**}**-O-[carbohydrate]**, where n = 1-10, including hydrocarbon isomers, ether linkage isomers and hydroxylated derivatives thereof;
   d. primary amine, aminoguanidine and guanidine derivatives of sucrose polyesters having one or more carbonyl trapping functional group per molecule wherein each carbonyl trapping functional group is in the omega-, omega-1 or other isomeric position within the fatty acyl chains, wherein each fatty acyl chain may have from 3 to 26 carbons, from one to five nitrogen functional groups and from one to 24 hydroxyl groups;
   e. synthetic polysaccharides consisting partly or entirely of aminosugars bound by beta-1,2, beta-1,3, beta-1,4 and/or beta-1,6 linkages;
   f. mixed polysaccharide polymeric derivatives wherein primary amine, aminoalkyl (one to ten carbons per alkyl group), amino-hydroxyalkyl (one to ten carbons per alkyl group and one to ten hydroxyl groups per alkyl group), aminoguanidine, aminoguanidinylalkyl (one to ten carbons per alkyl group), aminoalkyl-guanidinyl (one to ten carbons per alkyl group), guanidine, aminobenzene and/or aminoalkylbenzene (one to ten carbons per alkyl group) functional groups are covalently attached to matrices such as epi-chlorohydrin copolymers of cellulose or chitin and wherein hydrocarbon spacer groups may include alkene as well as alkyl groups; and
   g. non-polysaccharide polymeric derivatives wherein primary amine, aminoalkyl (one to ten carbons per alkyl group), aminohydroxyalkyl (one to ten carbons per alkyl group and one to ten hydroxyl groups per alkyl group), aminoguanidine, aminoguanidinylalkyl (one to ten carbons per alkyl group), aminoalkyl-guanidinyl (one to ten carbons per alkyl group), guanidine, aminobenzene and/or aminoalkylbenzene (one to ten carbons per alkyl group) functional groups are covalently attached to a synthetic non-digestible polymer selected from the group consisting of polystyrene, styrene-divinylbenzene copolymer, polyvinyl alcohol and crosslinked derivatives thereof, and wherein hydrocarbon spacer groups may include alkene as well as alkyl groups,
   in a dosage range of from one gram/day to 40 grams/day, in a microfibrillated form or microcrystalline form having enhanced surface area, increased porosity, increased water retention capacity and enhanced chemical accessibility, and in association with a pharmaceutically acceptable carrier thereof.

### BACKGROUND OF THE INVENTION

### 1. Field of the Invention

This invention relates to the use according to claim 1 for chronic inflammatory diseases, including chronic gingivitis, chronic periodontitis, chronic autoimmune gastritis, ileitis, colitis, interstitial cystitis, arthritis, tendinitis, carpel tunnel-syndrome and other cumulative trauma disorders, systemic lupus erythematosus, autoimmune vasculitis, asbestosis, silicosis, chronic obstructive pulmonary discase, Lyme disease, inflammatory myopathies, status epilepticus, inflammatory neuropathies, myasthenia gravis, as well as lessening of inflammatory site edema, and treatment of post-event ischemia and reperfusion symptomology resulting from acute central nervous system trauma, stroke and myocardial infarction excluding multiple sclerosis No pharmacological treatment of comprehensive effectiveness is currently available for any of the chronic inflammatory dis-orders or etiologically related symptomology discussed herein.

### 2. Description of Prior Art

The logic and potential value, even *synergistic* value, of using two or more therapeutic agents in combination has been recognized previously (Ghose and coworkers, 1983; Flood and coworkers, 1988; Goldstein and coworkers, 1990, pg. 102; Rinne, 1991). For example, Brooks and Schwarzer (1991) noted that:
Long term (five years or more) studies of SAARDs [slow acting antirheumatic drugs] in rheumatoid arthritis suggest that less than 50% of patients are taking gold, D-penicillamine, sulphasalazine, or anti-malarial drugs five years after the start.11-15 Patients whose disease was initially controlled by these agents ceased taking the drugs because of either the development of side effects or a flare in disease activity, despite continuation of the treatment...
...As the pathogenetic processes of rheumatoid arthritis are so complex it is extremely unlikely that any single agent will block all the pathways leading to joint destruction. It might, therefore, be reasonable to consider using combinations of drugs, either simultaneously or cyclically...

Numerous prior art publications disclose that vitamin E functions physiologically, as a lipid-soluble antioxidant free radical trapping agent. Prior art publications describe methionine as a water-soluble agent, an essential amino acid, an antioxidant and a free radical trapping agent.

Primary agents of this invention are selected from the group consisting of p-aminobenzoic acid (PABA) and derivatives thereof. p-Aminobenzoic acid is known as a water-soluble B vitamin, and several published studies have presented evidence to the effect that PABA functions, in part, as a weak anti-oxidant and a weak free radical trapping agent (Maksimov and Rebachuk, 1985, Table 2; Pryor and coworkers, 1976, pg. 201).

In so far as benzoic acid or derivatives thereof have been recognized as antioxidants or free radical trapping agents, their mechanism of action is understood to consist of hydroxyl radical trapping by the benzene ring (Grootveld and Halliwell, 1988; Halliwell and Gutteridge, 1985, pp. 105 and 130; Richmond and coworkers, 1981; Repine and coworkers, 1979, pg. 1642). This has been explicitly demonstrated for PABA (Nakken, 1964, pp. 446, 448, 454-457; Nakken and Pihl, 1966, pp. 21, 22, 24, 25 and 28). Likewise, mannitol is recognized as an antioxidant based on its ability to scavenge hydroxyl radicals (Halliwell and Gutteridge, 1985, pp. 97 and 105), and a similar mechanism of action is recognized for dimethyl sulfoxide (Halliwell and Gutteridge, 1985, pg. 147) and methionine (Del Maestro, 1980, 164-165).

Clinical use of the drug sulfasalazine (SAZ) represents a well documented example of the use of a benzoic acid derivative as a trapping agent for the hydroxyl radical and other free radicals in the treatment of a chronic inflammatory disease. In the colon SAZ undergoes reductive cleavage to liberate 5-amino-salicylic acid (5-ASA), which is the therapeutically active agent. Ahnfelt-Ronne and coworkers (1990) have presented research findings which docoment the use of sulfasalazine for successful treatment of chronic inflamatory bowel disease (CIBD), also known as ulcerative colitis. Summarizing their work, these investigatons noted:
The present results thus lead us to propose the following mechanism of action of SAZ in the treatment of CIBD. After cleavage of SAZ by reductive processes in the colon, 5-ASA is released in high concentrations. 5-Aminosalicylic acid is a highly effective scavenger of free radicals and interacts with hydroxyl and other radicals to form a novel major metabolite of 5-ASA, M1 and several minor metabolites. In this reaction, 5-ASA breaks the free radical chain reaction. The cytotoxic and tissue-destructive effects of this otherwise perpetuating process are thereby gradually aborted. This action of 5-ASA manifests itself clinically as an antiinflammatory effect, and the proposed mechanism of action implies that free radicals are of major importance in producing the clinical signs of chronic inflammation...(pgs. 1168-1169)

Yet several points serve to distinguish this previous work on SAZ from the present invention. Based on high performance liquid chromatographic elution times and ultraviolet spectra data, Ahnfelt-Ronne compared their in vivo 5-ASA metabolic products to products observed after in vitro hydroxylation of 5-ASA by the Fenton reaction and tentatively identified 5-ASA metabolites as being hydroxylated derivatives. Yet they never explicitly identified the chemical structures of the disease-specific metabolic products they observed. Nor was there any attempt on their part to look for evidence of in vivo trapping of carbonyl products. Dull and coworkers (1987, pg. 2469) used mass spectrometry to definitively identify two of the several hydroxylation/oxidative deamination products which result from in vitro incubation of 5-ASA with activated human mononuclear cells. They identified these products as gentisic acid (2,5-dihydroxybenzoic acid) and salicylic acid (2-hydroxy benzoic acid), while five other 5-ASA metabolic products remained unidentified (pg. 2470).

There also was a curious methodological oversight in the study of Ahnfelt-Ronne and coworkers. They included several rheumatoid arthritis (RA) patients in their SAZ study as one of their control groups and reported, in part:
The presence of M1 and other Fenton reaction products of 5-ASA in extracts of feces samples from CIBD patients treated with SAZ is clearly demonstrated.
In contrast, none of the metabolites M1-M6 were detected in stool extracts from SAZ-treated RA patients. These extracts contained 5-ASA, sulfapyridine, and N-acetyl-5-ASA in the normal range for SAZ-treated patients...(pg. 1165)

This represents a misconceived use of a control group, as CIBD is a chronic inflammatory disorder of the intestinal tract, while RA is not. Ahnfelt-Ronne and coworkers failed to examine urine or blood samples from their RA patients for evidence of metabolites M1-M6, which is where one would logically expect to find such metabolites.

Ahnfelt-Ronne and colleagues, like earlier investigators, never recognized the possibility of using a therapeutic agent to scavenge carbonyl products of inflammation. Hence they never recognized the possibility of intentionally using a composition consisting of a primary agent which sequesters carbonyl products in combination with co-agents that have known antioxidant properties.

Further distinctions should be noted between the present invention and previously recognized clinical use of SAZ in that SAZ releases sulfapyridine, a somewhat toxic substance, into into the body (Peppercorn, 1984, pgs. 377-379 and 383), while the present invention does not, and use of sulfasalazine depends on intestinal bacteria for activation of the drug, while the primary agents of the present invention do not. Besides use in treatment of CIBD and ileitis (Budavari and coworkers, 1989, pg. 1412), SAZ has been recognized , at least at the experimental level, for treatment of radiation bowel disease, scleroderma, dermatitis herpetiformis and rheumatoid arthritis (Peppercorn, 1984, pgs 380-381).

Other examples of amine drugs recognized as having anti-inflammatory properties include para-substituted N-benzenesulfonyl derivatives of anthrilic acid (Borne and coworkers, 1974), 4-amino benzoic acid anilides (Thiele, 1971; Deutsche Gold- und Silber-Scheideanstalt vorm. Roessler, 1972) and Tinoridinc (Shimada and Yasuda, 1979). The chemical structures of these agents lie beyond the present invention, they are not presently recognized as carbonyl sequestering agents, and they have not been used in multiple ingredient compositions analogous to those of the present invention.

Several drug products containing PABA have been marketed for human use in the United States. However, none include the inventive concept or compositions claimed herein. Potassium p-aminobenzoate has been marketed as *Potaba* (*R*) in the pure form as an antifibrotic, i.e., skin softening, agent (Drug Information for the Health Care Professional, 8th ed., 1988, pgs. 111-113). As such it has been recognized for treatment of Peyronie's disease; diffuse systemic scelerosis; morphea and linear scleroderma; and dermatomyositis. For such purposes, *Potaba* (R) is taken orally in average doses of 12 grams/day for up to two years, although human use of 15 - 20 grams/day is recognized. As an ingredient in analgesic tablets, PABA has been marketed for domestic human use (300 mg/tablet) in *Pabirin* (R) buffered tablets (with aspirin), in *Pabalate (R)* tablets (with sodium salicylate) and in *Pabalate-SF (R)* tablets (with potassium salicylate), as described in Physicians' Desk Reference (Huff, 1980, pgs. 849, with aspirin and 1430, with salicylates). Five percent PABA in a cream base has also been marketed as a sunscreen product (Physicians' Desk Reference, Huff, 1980, pg. 849).

In its summary on systemic use of PABA the Drug Information for the Health Care Professional text (8th ed., 1988, pg. 111) presented the following statement (reproduced herein its entirety):
Mechanism of action: The mechanism by which aminobenzoate potassium exerts its antifibrotic effect is not known. It has been postulated that fibrosis results from an imbalance of serotonin and monoamine oxidase (MAO) mechanisms at the tissue level. Fibrosis is believed to occur when an excessive serotonin effect is sustained over a period of time. This could be the result of too much serotonin or too little MAO activity. Aminobenzoate potassium increases oxygen utilization at the tissue level. It has been suggested that this increased oxygen utilization could enhance the degradation of serotonin by enhancing MAO activity or other activities that decrease the tissue concentration of serotonin.

In its summary on systemic use of potassium p-aminobenzoate the Physician's Desk Reference (Dowd, 1993, pg. 1103) presented the following statement (reproduced herein its entirety):

### INDICATIONS

Based on a review of this drug by the National Academy of Sciences-National Research Council and/or other information, FDA has classified the indications as follows:
'Possibly' effective: Potassium aminobenzoate is possibly effective in the treatment of scleroderma, dermatomyositis, morphea, linear scleroderma, pemphigus, and Peyronie's disease.
Final classification of the less-than-effective indications requires further investigation.

### ADVANTAGES

POTABA offers a means of treatment of serious and often chronic entities involving fibrosis and nonsuppurative inflammation.

### PHARMACOLOGY

P-Aminobenzoate is considered a member of the vitamin B complex. Small amounts are found in cereal, eggs, milk and meats. Detectable amounts are normally present in human blood, spinal fluid, urine, and sweat. PABA is a component of several biologically important systems, and it participates in a number of fundamental biological processes. It has been suggested that the antifibrosis action of POTABA is due to its mediation of increased oxygen uptake at the tissue level. Fibrosis is believed to occur from either too much serotonin or too little monoamine oxidase activity over a period of time. Monoamine oxidase requires an adequate supply of oxygen to function properly. By increasing oxygen supply at the tissue level POTABA may enhance MAO activity and prevent or bring about regression of fibrosis.

This inventor sees no relationship of such comments to the present invention. In particular, the comments noted above clearly do not recognize the potential use of PABA and derivatives thereof as carbonyl trapping agents, that is, as agents which may *generally* inhibit chronic inflammatory disorders by virtue of their ability to chemically bind to and sequester aldehyde and ketone products of lipid peroxidation which result from and contribute to the continuation of chronic inflammatory disorders. Hence the clinical applications of PABA and derivatives thereof claimed in the present invention are regarded by the inventor as new and novel.

Certain amine agents have recognized antioxidant properties. These include N,N'-di-(sec-butyl)-p-phenylenediamine (Scott, 1965, pg. 120), aniline and aniline N-subsyituted agents (Scott, 1965, pg. 125). In the present invention focus is placed on primary amine agents, as such agents are known to covalently react with carbonyl agents to yield Schiff base-type products (Feeney and coworkers, 1975, pg. 141). By contrast, N-substitution with hydrocarbon functional groups tends to increase amine antioxidant activity (Scott, 1965, pgs. 125 and 148). These are two distinct chemical phenomena. The antioxidant property of amines depends on their ability to act as electron donors to alkoxy or alkylperoxy radicals (Scott, 1965; pgs. 127, 145 and 158). The carbonyl trapping property of amines depends on their ability to form Schiff base-type addition products.

Vitamin C (ascorbic acid) is widely recognized as a water-soluble antioxidant vitamin. However, numerous published studies which have appeared since 1980 document that vitamin C also can act physiologically as a pro-oxidant (Gutteridge and Wilkins, 1982), an agent which stimulates lipid peroxidation (Chojkier and coworkers, 1989, pgs. 16957 and 16961), and that it is a strong protein glycosylating agent (Ortwerth and Olesen, 1988, pgs. 12, 14, 16, 18 and 20). Thus, for example, in vitro studies have documented the ability of vitamin C to accelerate the process of cataract formation (Slight and coworkers, 1990, pgs. 369-373). In addition, some evidence suggests that ascorbic acid may act as a factor which stimulates certain reactions which are characteristic of inflammatory diseases. For example, the presence of ascorbic acid in the synovial fluid of the arthritic joint may contribute to degradation of hyaluronic acid (Wong and coworkers, 1981; Higson and coworkers, 1988).

The use of vitamin B₆ as a primary agent in combination with co-agents such as vitamins C, E, A, B₁, B₅, as well as dimethyl sulfoxide, PABA, inositol, selenium, butylated hydroxytoluene, thiodipropionates, choline, cysteine, zinc and D-penicillamine has been described for clinical treatment of arthritis, together with use of a water bed (Pearson and Shaw, 1982, pp. 298-300). Numerous other variations on this list of co-agents have been described publicly (see Passwater, 1985).

The disclosure of Pearson and Shaw contains several deficiencies which are resolved by the present invention. They did not recognize the pro-oxidant, the lipid peroxidation stimulating or the protein glycosylating properties of vitamin C. Noting these deleterious properties of vitamin C, this inventor regards excess vitamin C consumption (beyond the RDA. 60 mg/day) to be a risk factor for exasperating the physiological and clinical effects of arthritis and other chronic inflammatory diseases. In contrast, Pearson and Shaw (1982) refer repeatedly to vitamin C as the primary agent of their many compositions (for example, pgs. 468-469 and 611-613).

Nor did Pearson and Shaw recognize the ability of aldehyde trapping agents such as PABA to chemically react with and sequester aldehydes which may result from and contribute to the inflammatory cascade process. This inventive oversight on their part is of fundamental importance. This explains why Pearson and Shaw listed PABA only as one of many "anti-oxidant" and "membrane stabilizer" co-agents. In contrast, PABA and derivatives thereof are identified as the class of primary agents in the present invention.

At least two other deficiencies of the Pearson and Shaw prior art disclosure are addressed by the present invention. D-Penicillamine has been recognized as an anti-arthritic drug based on its abilities to inhibit lipid peroxidation by neutralization of free radicals and to bind iron or copper ions (Kostyuk and coworkers, 1990, pg. 39; Demopoulos, 1973, pgs. 1904 and 1906). D-Penicillamine has known predictable long term toxic properties which severely limit its practical use on humans (Demopoulos, 1973, pg. 1906; Zuckerman and coworkers, 1980, pg. 430). Its toxic properties prevent its use on a long term, on-going basis, as required for the clinical treatment of the disorders addressed herein. In addition, L-cysteine has been recognized as a neurotoxic agent when used under certain laboratory circumstances (Olney and coworkers, 1990). As Olney and coworkers stated, "after systemic administration to immature rodents, L-cysteine destroys neurons in the cerebral cortex, hippocampus, thalamus, and straitum..." (pg. 596). Hence use of cysteine is also deleted from the present invention.

Zarafonetis (1953) has reported some success in treatment of rheumatoid arthritis by use of potassium p-aminobenzoate in combination with acetylsalicylic acid and cortisone. In this report Zarafonetis also described some success in clinical treatment of dermatomyositis and scleroderma by use of potassium p-aminobenzoate alone, and referred to earlier work on these disorders and other clinically related syndromes, including forms of lupus erythematosus.

Yet Zarafonetis based his logic for diversifying clinical studies on PABA or its potassium salt solely on similarities of clinical symptoms, comparisons among clinical syndromes which feature some common symptomology (Zarafonetis, 1953, pp 667-668; Zarafonetis, 1964, pgs. 550 and 560; Priestley and Brown, 1979, pg. 161; Zarafonetis and coworkers, 1988, pg. 194). Zarafonetis never stated an understanding or recognized that PABA has the physiological potential of serving as an aldehyde chemical trapping agent (Zarafonctis, 1953, pg. 671). Hence, he never recognized its potential to sequester aldehyde products resulting from increased lipid peroxidation secondary to site-specific inflammation. In failing to recognize this principle, Zarafonetis failed to recognize the potential full scope of clinical applications of PABA. Failing to recognize the potential of synergistic antioxidant co-agents, the procedures of Zarafonetis for treatment of scleroderma, rheumatoid arthritis and dermatomyositis relied on use of high PABA dosages (12-18 gm/day; sec Zarafonetis, 1953, pg. 666). In principle, it is the understanding of this inventor that the clinical prognosis of any disease which features increased lipid peroxidation as part of its etiology may be improved by clinical application of the present inventive concept.

Zarafonetis (1953) also referred to an earlier study which used a combination of p-aminobenzoic acid and alpha-tocopherol to treat scleroderma. Gougerot and Hewitt (1951) described the logic of their scleroderma treatment protocol as follows:
This observation is to be added to the file of the treatment of sclerodermas. Zarafonetis and his collaborators have already published 5 cases of sclerodermas improved by para-aminobenzoic acid, and in the same therapeutic series Shaffer and his collaborators treated a gener alized scleroderma with para-aminobenzoic acid with improvement. In a study of a completely different nature, vitamin E (a-tocopherol) was used by Klemperer, etc. and in France by Bazex (Lyon, July 1949). This is why we have associated the two therapies because of their effect on diseases of collagen.

As such, they perceived their clinical treatment strategy to address the status of collagen, with no discussion of possible physiological mechanisms. Compared to the present invention, Gougerot and Hewitt:
(1) failed to recognize that either of their therapeutic agents may interfere with the inflammatory cascade,
(2) failed to recognize that primary amine and amine-related derivatives of benzoic acid, as a class, may bind to and sequester aldehydes which result from the inflammatory process, and
(3) failed to understand that the combination of a water soluble aldehyde-trapping primary amino agent and a lipophilic antioxidant agent may have clinical application to the treatment of a broad spectrum of chronic inflammatory diseases.

One additional clinical study based on use of PABA falls within the scope of prior art regarding the present invention. In 1967 Mel'nikova and Ryzhova presented the results of a clinical trial wherein PABA was used to treat post-event trauma in experimental myocardiual infarction, as studied in rabbits and dogs. Myocardial infarction was induced by silk ligature of the left coronary artery and vein. Mel'nikova and Ryzhova reported, in part:
One of the more important problems facing modern pharmacology is the search for substances capable of restoring the circulation of the heart in myocardial infarction. Improvement in the coronary circulation may be obtained both by a direct coronary-dilator effect on the vessels of the heart and by influences acting on metabolic processes and the hemodynamics in the heart muscle. From this point of view there is considerable interest in procaine and the product of its hydrolysis - p-aminobenzoic acid (PABA), an active component of procaine, taking part in intimate biochemical processes and possessing a well-defined antihistamine action...(pg. 389)
The animals of series II (25 rabbits and 5 dogs) with an acute myocardial infarct received subcutancous injections of PABA in doses of 12-15 mg/kg daily, twice a day throughout the period of observation. Examination of the electrocardiographic, clinical, and laboratory findings for the rabbits and dogs treated with PABA (12 mg/kg) on the second day showed restoration of the normal rhythm with disappearance of the extrasystoles, an increase in the voltage of the waves, and some reduction in the depth of the QS waves, evidently as a result of improvement of the coronary circulation...On the 9th-14th day, in 85% of the animals (receiving 12 mg/kg PABA) the ECG and results of the clinical and laboratory investigations were normal, while in the remaining 15% of animals, organization of the myocardial infarct took place on the 16th-18th day...(pg. 390)
Special attention was drawn to the well marked vascularization both in the areas of connective scar tissue and throughout the myocardium, mainly as a result of dilatation of the capillaries and an increase in the number of arteries, and also to the absence of necrosis in the perifocal zone.
In acute experiments on cats (by N. V. Kaverina's method) a constant,and well marked coronary-dilator effect was observed in response to injection of PABA (15-20 mg/kg), as shown by a considerable increase in the volume velocity of the coronary blood flow (by 80-108%) and also by a distinct reduction in the oxygen consumption of the heart...(pg. 391)

As such, Mel'nikova and Ryzhova have reported a coronary vasodilator effect of PABA based on their understanding of the drug's antihistamine property. They recognized no anti-inflammatory property of PABA and did not understand their findings within such a context. Subsequent work by Kurdin (1978) has presented evidence of increased lipid peroxidation in the process of myocardial infarction. Viewing both of these studies within the context of the present invention, the inventor proposes that, to some degree, the beneficial effects of PABA in the Mel'nikova and Ryzhova study reflected an anti-inflammatory property of PABA unrecognized by the investigators, and that such a beneficial effect may be optimized by use of the multicomponent compositions of the present invention. As defined below, the present invention is believed to have significant clinical value in the post-event treatment of myocardial infarction, acute central nervous system trauma and stroke.

United States Patent No. 5,002,703 (Warner-Lambert Co.) is entitled "Benzoic acid and benzoic acid ester derivatives having antiinflammatory and analgesic activity." It's utility is described as applying to "...treatment of arthritis, asthma, Raynaud's disease, inflammatory bowel disorders, trigeminal or herpetic neuralgia, inflammatory eye disorders, psoriasis, dental pain, and headaches, particularly vascular headache, such as migraine, cluster, mixed vascular syndromes, as well as nonvascular, tension headaches." The mechanism of action as defined in this patent is different from the inventive concept of the present invention, the primary agents of the Warner-Lambert procedures are substantially different from those of the present invention, and the Warner-Lambert procedures do not involve multiple-ingredient compositions analogous to those of the present invention. All Warner-Lambert US patents of the series represented by US Patent 5,002,703 are based on claims regarding compounds which are, by comparison to the present invention, relatively large, complex chemical derivatives of benzoic acid. For example, the primary agents of US Patent 5,002,703 are described as:
A compound or the formula

   R₁, (Q-R-B-N(-Y)-CH(-X)-)benzene
wherein: (a) R₁ is COOR' wherein R' is H or lower alkyl of one to four carbons, inclusive; (b) B is -SO₂-; (c) X and Y arc independently H or lower alkyl of one to four carbons, inclusive; (d) R₂ is alkylene, alkenylene, alkynylene branched or linear chains of 1 to 11 carbons, inclusive; (c) Q is COOH, Br, NH₂, cyclohexyl, or 2-naphthyl-O-; or nontoxic, pharmaceutically acceptable acid addition or base salt thereof.

Chemical structures of this kind clearly lie beyond the claims of the present invention.

Broad spectrum clinical use of anti-inflammatory vitamin compositions which feature PABA as a primary agent, and the methodological reasoning for doing so, has not been previously recognized or described, p-Aminobenzoic acid is not presently recognized as a non-steroidal anti-inflammatory drug (NSAID). Hence, for example, it is not included in the lists of such drugs published in (a) the Merek Index, (Budavari, 1989, pages THER-15 to THER-16), (b) Scientific American (Wcissmann, 1991, page 86), and (c) Understanding Arthritis (Kushner, 1984, pages 52-53). Likewise, p-aminobenzoic acid is not recognized as being a "slow acting" anti-inflammatory agent (Understanding Arthritis, Kushner, 1984, pages 55-57). Indeed, in a feature article entitled "The new scoop on vitamins," Time magazine (Toufexis, 1992) failed to make any reference whatsoever to PABA.

### SUMMARY OF THE INVENTION

These and other objects of this invention are achieved by providing a use according to claim 1.

This invention involves use of orally administered amine and amine-related derivatives of benzoic acid as carbonyl trapping agents. These primary therapeutic agents act by chemically binding to and sequestering the aldehyde and/or ketone products of lipid peroxidation. Increased levels of lipid peroxidation have been repeatedly demonstrated as a part of the "inflammatory cascade" process which underlies the secondary etiology of chronic inflammatory diseases.

p-Aminobenzoic acid is an example of the primary absorbable pharmacological agent of the present invention. PABA has a small molecular weight, is water soluble, has a primary amine group which should react with carbonyl-containing matabolites under physiological conditions and is tolerated by the body in relatively high dosages and for extended periods. The present invention sets forth the belief that carbonyl sequestering agents administered in oral dosages may be used in combination with co-agents consisting of proven antioxidant free radical trapping agents, and agents related thereto, so as to produce a synergistic physiological effect of an anti-inflammatory and analgesic nature. The co-agents are defined in claim 1.

### 1. Aims of the Invention

Accordingly, it is a general object of this invention to treat the symptomology of chronic inflammatory diseases and etiologically related symptomology by use of carbonyl trapping agents in combination with known antioxidant free radical trapping co-agents and factors related thereto, so as to create compositions with additive, complimentary physiological therapeutic characteristics. It is a further object of this invention to facilitate the effectiveness of this anti-inflammatory and analgesic procedure by use of orally consumed carbonyl trapping agents which are of a non-absorbable nature, so as to bind and sequester carbonyl chemical agents which are present in food, thus preventing such toxic agents from being absorbed into the body.

It is an object of the present invention that the drug compositions described herein may provide clinical value in the treatment of disease symptomology for disorders featuring lipid peroxidation and resultant formation of toxic carbonyl compounds, including: chronic gingivitis, chronic periodontitis, chronic autoimmune gastritis, ileitis, colitis, interstitial cystitis, arthritis, tendinitis, carpel tunnel syndrome and other cumulative trauma disorders, systemic lupus erythematosus, autoimmune vasculitis, asbestosis, silicosis, chronic obstructive pulmonary disease, chronic obstructive pulmonary disease, Lyme disease, inflammatory myopathies, status epilepticus, inflammatory neuropathies, myasthenia gravis,
as well as lessening of inflammatory site edema, and post-event ischemia and reperfusion injury resulting from acute central nervous system trauma, stroke, kidney ischemia or myocardial infarction; excluding multiple sclerosis.

This object is achieved by the use of a pharmaceutical composition for the manufacture of a medicament in accordance with claim 1.

It is another object of the present invention that in so far as the therapeutic procedures described herein may serve to delay the necessity of initiating the use of known medicaments or to decrease the dosages of known medicaments required to achieve beneficial effects, the period of prior art drug therapeutic value may be extended and detrimental clinical side effects resulting from use of known medicaments may be decreased, so that overall patient treatment may be improved.

It is a further object of this invention that the absorbable amine and amine-related substances and derivatives thereof described herein when used in combination with specified co-agents may be clinically applied to treat veterinary disorders comparable to at least some of those human disorders described above.

### 2. Statement of Invention

It is known that aldehyde chemical metabolites, which contain carbonyl functional groups, are generated during the process of chronic inflammation. These aldehyde products result from pathologically increased lipid peroxidation, which may be initiated by a variety of activated oxygen chemical species such as the hydroxyl radical, HO⁻ (Halliwell and Gutteridge, 1985, pp. 119-120). The reactive cascade of free radical propagation → lipid peroxidation → aldehyde formation and other subsequent effects of inflammation is well documented in the prior art (Halliwell and Gutteridge, 1985, pp. 102-103).

The concept of using carbonyl-trapping agents such as primary amine or amine-related derivatives of benzoic acid to treat chronic inflammatory diseases has not been recognized or disclosed. Thus, the application of this principle in conjunction with use of known antioxidant free radical trapping agents to produce new and novel compositions which have improved, synergistic therapeutic properties also has not been recognized. When compared to previously disclosed understanding of the actions of recognized non-steroidal anti-inflammatory drugs (Weissmann, 1991), it is evident that the inventive concept described herein represents a new approach to the treatment of chronic inflammatory disorders.

Previously, attempts at pharmaceutical intervention in this cascade of inflammatory reactions has focused primarily on use of both water-soluble and lipid-soluble antioxidant free radical trapping agents or use of metal chelating agents (Halliwell and Gutteridge, 1985, pp. 125 and 116-117). As iron and copper ions have been shown to induce hydroxyl radical formation (Halliwell and Gutteridge, 1985, pg. 123) and induce lipid peroxidation (Halliwell and Gutteridge, 1985, pg. 124), the use of metal chelating agents such as desferoxamine to ameliorate the inflammatory cascade has received some attention (Halliwell and Gutteridge, 1985, pp. 116-117). However, desferoxamine has predictable ocular and auditory side effects (Halliwell and Gutteridge, 1985, pgs. 117 and 140), and present examples of anti-oxidant free radical trapping agents and combinations thereof have proven to be of limited clinical value.

Both PABA and penicillamine are primary amine agents which also function as antioxidant free radical trapping agents. Yet as antioxidant agents PABA and penicillamine are presently regarded as being of secondary, nominal value, due either to weak anti-oxidant properties or toxic side effects, respectively. Thus their use as anti-inflammatory agents has been quite limited. Their potential value for trapping the aldehyde products of inflammation-related lipid peroxidation has never been recognized. Hence the formulation of a new composition, such as one having PABA as its primary agent, known antioxidant free radical scavenging chemicals as co-agents and lacking vitamin C, has never been previously described, and the potential for clinical use of such a novel composition in treatment of chronic inflammatory diseases never recognized.

Further distinctions should be made between the present invention and previously recognized use of penicillamine, one of the "slow-acting" anti-inflammatory drugs mentioned in Understanding Arthritis (Kushner, 1984), a publication of the Arthritis Foundation. The primary amine and amine-related principle agents described in the present invention are all derivatives of aminobenzoic acid, which should facilitate their safe climination from the body by normal kidney filtration . Penicillamine is not a derivative of aminobenzoic acid. In addition, penicillamine has a reduced sulfhydryl group, unlike any of the primary agents claimed herein. However, penicillamine does have a primary amine functional group as well as a carboxylic acid functional group, like aminobenzoic acid. In Understanding Arthritis Kushner noted that:
Many doctors believe that the slow-acting drugs may slow the underlying disease, though how they do this is not clear. This group of drugs includes gold, penicillamine, cytotoxic, and antimalarial drugs. All of the drugs in this group have to be taken for many weeks, and often for several months, before their full effects become noticable. The relief they provide may last for some time after they are no longer being taken. But with these benefits of long-lasting relief and a possible slowing of the disease also comes a higher risk of serious side effects... (pages 55-56) ...Again, the side effects [of penicillamine] often require some people to stop taking this drug. Like gold, penicillamine may damage the kidneys and bone marrow, and may also cause fever, chills, rashes, sores in the mouth, a sore throat, stomach upset, muscle weakness, loss of taste, and easy bruising or bleeding. Because of these possible side effects, the drug is taken only with close supervision by a doctor...(page 57)

The present invention constitutes an alternative slow-acting anti-inflammatory composition which is believed to be inherently safer for the patient and act via a mechanism not previously recognized or described.

A review of the article by Weissmann (Scientific American, 1991, pages 84-90) will serve to illustrate the distinction between the present invention and prior art development of non-steroidal anti-inflammatory drugs. In discussing presently recognized understanding of the actions of NSAIDs, Weissmann stated, in part:
NSAIDs act by blocking the production of both PGG and PGH₂. Platelets transform the latter into a most potent vasoconstricting and platelet-aggregating substance, thromboxane B₂...(page 87)
...Aspirin irreversibly inactivates PGH synthase [i.e., prostaglandin H synthase]. Platelets cannot make more synthase and so make no more thromboxane...less than one tablet a day can irreversibly block the PGH synthase activity of platelets...(page 88)
Vane's theory that the local production of prostaglandins leads to inflammation has only partly been substantiated, however...aspirinlike drugs exert clinical effects that do not depend on inhibiting prostaglandin synthesis...(page 88)
The broad spectrum of NSAID effects most likely results from their physical properties, which permit them to disrupt interactions within biological membranes... For example, aspirin alters the uptake of fatty acids and their insertion into the membranes of cultured human monocytes and macrophages. Salicylates also inhibit anion transport across a variety of cell membranes. Finally, NSAIDs inhibit bone metabolism and the synthesis of proteoglycan, a substance that forms the matrix of cartilage...(page 88)
Recent work in my laboratory has uncovered an alternative mechanism for the effects of aspirinlike drugs: interference with stimulus-response coupling in neutrophils, the most abundant cells of acute inflammation. These cells are the first line of defense against foreign intruders and among the first to cause injury in autoimmune diseases like rheumatoid arthritis. They damage tissues by releasing enzymes that break down proteins (proteases), as well as inflammatory peptides, reactive oxygen species such as O₂ and H₂O₂ (peroxide), and lipid irritants such as platelet-activating factor and leukotriene B₁. (pages 88-89)
...Both 'homotypic' adhesion between neutrophils and 'heterotypic' adhesion of neutrophils to the walls of blood vessels are required for cells to make their way out of the circulatory system and to cause inflammation... It is therefore likely that the anti-inflammatory effect is related to the ability of both [sodium salicylate and aspirin] compounds to inhibit homotypic and heterotypic adhesion in neutrophils rather than to their unequal effect on prostaglandin synthesis...
All NSAIDs inhibit the homotypic adhesion of neutrophils, but they differ in their effects on other functions of the neutrophil...(page 89)
Some of the effect of NSAIDs on cells arises from interference with the binding of chemoattractants and other stimuli. These drugs inhibit the binding of at least some of these ligands with their receptors in the cell membrane...NSAIDs interfere in particular with signals that depend on so-called G proteins for transduction through cell membranes. (page 90)

The present invention, by contrast, depends on a fundamentally different mechanism of action. The present invention is based on use of primary amine or amine-related derivatives of benzoic acid as principle agents for chemically binding to and sequestering aldehyde products of inflammation site lipid peroxidation in combination with previously recognized antioxidant free radical trapping co-agents. This unique, multiple-ingredient approach to interfer with certain steps in the inflammatory cascade has not been previously recognized by other research investigators.

This is, in fact, the first anti-inflammatory agent invention which addresses the issue of aldehyde formation at inflammation sites. As aldehydes are highly reactive molecules capable of reacting with proteins, lipids and nucloic acids (Jollum and coworkers, 1973, pg. 200; Carden and coworkers, 1986), their increased formation at inflammation sites may be a significant contributing factor in the evolution of the clinical pathology of inflammatory disorders. Halliwell and Gutteridge (1985, pg. 123) noted that malonaldehyde
...is only one of a great number of carbonyl compounds formed in peroxidising systems and often is only a tiny percentage of the total products formed...Other toxic aldehydes include 4,5-dihydroxydecenal and 4-hydroxynonenal. Lipid peroxides and/or cytotoxic aldchydes derived from them can block macrophage action, inhibit protein synthesis, kill bacteria, inactivate enzymes, crosslink proteins and generate thrombin...

The results of several published research studies suggest that dysfunctional lipid peroxidation may be a contributing factor in the etiology of a variety of chronic inflammatory diseases, such as rheumatoid arthritis (Rowley and coworkers, 1984), multiple sclerosis (Hunter and coworkers, 1985), silicosis (Katsnelson and co-workers, 1989, pg. 318), Duchenne muscular dystrophy (Kar and Pearson, 1979; Jackson and coworkers, 1984), and chronic inflammatory bowel disease (Ahnfelt-Ronne and coworkers, 1990). As exposure to asbestos fibers can stimulate lipid peroxidation (Halliwell and Gutteridge, 1985, pg. 152), asbestosis should also be included in this category. Increased lipid peroxidation has also been demonstrated in acute central nervous system trauma (Hall, 1987, pgs. 421 and 424; Demopoulos and coworkers, 1980, pg. 97; Kontos and coworkers, 1981, pg. 2329), as a result of stroke (Zivin and Choi, 1991, pg. 61) and subsequent to myocardial infarction (Kurdin, 1978). Status epilepticus is one of several clinical disorders which have been linked to increased intracellular concentrations of free radicals, with subsequent lipid peroxidation (Del Maestro, 1980, pg. 163). Likewise, published evidence has documented the ability of carbonyl compounds resulting from lipid peroxidation to induce foot edema in the rat (Benedetti and coworkers, 1980).

### (3) Detailed Description of the Preferred Embodiment

The inventive feature disclosed in this text is that compositions consisting of absorbable carbonyl trapping drugs in combination with known antioxidant free radical trapping co-agents may be of particular synergistic benefit in preventing or ameliorating forms of chronic inflammation by incorporating two pharmacological strategies, the sequestering of cytotoxic aldehydes and ketones generated at sites of chronic inflammation and the sequestering of activated oxygen chemical species generated earlier in the inflammatory cascade. It is understood that oral use of non-absorbable carbonyl trapping agents may serve to prevent absorption of dietary aldehydes and ketones from the alimentary tract into the body, thus complementing the intended therapeutic results.

### (i) Mechanism of Action of Primary Agents

For the most part, these pharmacological reactions are based on the ability of primary amine compounds to react with aldehyde functional groups of potentially toxic agents, yielding covalently bound Schiff base products. Several examples of chemically analogous reactions, presented within other contexts, have been publicly presented. Representative examples are discussed below. These model chemical systems are directly analogous to the proposed mechanism of drug action which is the basis of the present invention.

Comments by Feency and coworkers (1975, pg. 141) provide an appropriate introduction to this subject:
A wide variety or substances with -NH₂ groups condense with carbonyl compounds ... This condensation of primary amines with aldehydes and ketones to give imines was first discovered by Schiff (1900). The overall equilibrium greatly favors hydrolysis in aqueous solution for aliphatic aldehydes. With aromatic aldehydes, the equilibrium is shifted in favor of Schiff base formation. It is important to note that increasing the nucleophilic strength of the amine will increase the rate of the carbonylamine reaction but will have almost no effect on the position of the equilibrium.

These comments suggest that the amine-containing carbonyl-trapping drugs described herein should have particular promise for binding furanaldehydes, which are aromatic. These comments also suggest that doses of absorbable amine drugs may require in vivo concentrations in the range of 1:100 to 1:1,000 (carbonyl: amine) in order to achieve clinical effectiveness. This, in turn, suggests that therapeutic dosages may lie in the range of grams per day and that only drugs of particularly low toxicity will have human applications.

Feeney and coworkers (1975, pg. 144) also noted the phenomenon of Schiff base transimination, which occurs to a significant extent at neutral pH:

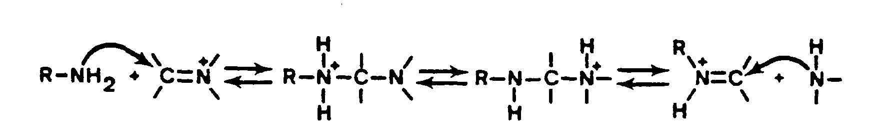

The existence of such non-enzymatic reversible transimination reactions is important within the context of this invention, as it suggests that in vivo both bound carbonyl agents, in addition to free carbonyl agents, may be sequestered by amine-containing drugs.
(a) The direct in vitro addition of p-aminobenzoic acid or ethyl p-aminobenzoate to malondialdehyde or its tautomer, beta-hydroxyacrolein, has been described (Sawicki and coworkers. 1963). 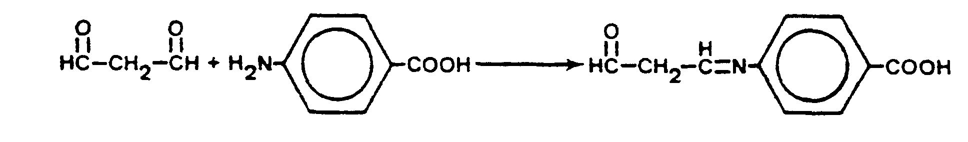
   This report also described the reaction of aminobenzene (that is, aniline) with malonaldehyde. The metabolic fate of PABA in humans has been actively investigated and well reported in the biomedical literature (Young and coworkers, 1971; Howie and Bourke, 1979). It is so actively metabolized via several mechanisms and quantitatively removed in urine (Weizman and coworkers, 1985; Bingham and Cummings, 1983) that PABA excretion has become a widely recognized standard for measuring urinary clearance. Small amounts of PABA are normally present in the human diet. It is recognized as being a vitamin for many organisms and is classified as a member of the vitamin B complex (Smith, 1976, pg. 194; Winitz and coworkers, 1970, pgs. 527-528; Scott and Robbins, 1942). As a vitamin for human use PABA is commercially marketed in the United States in the dosage range of 5 to 550 mg/day.
(b) Self-polymerization of o-aminobenzaldehyde has been described. In the 1993 edition of the Sigma Chemical Company catalog of biochemical reagents the following statement appears on page 87 of its listing: "**o-AMINOBENZALDEHYDE Unstable!** [store at] -20°C Polymerizes rapidly when exposed to room temperature. May yield slightly hazy solution in ethanol due to presence of a small amount of polymer. Shipped in dry ice." This information directly indicates that a primary amino group covalently linked to a benzene ring possesses sufficient reactivity for significant reaction with aldehyde functional groups at room temperature. It is apparent that no form of activation of the amino group is required and that a Schiff base product forms readily.
(c) The direct in vitro addition of n-hexylamine to beta-hydroxyacrolein to produce an N,N'-disubstituted 1-amino-3-iminopropene derivative has been reported (Chio and Tappel, 1969). The reaction may be represented as follows: 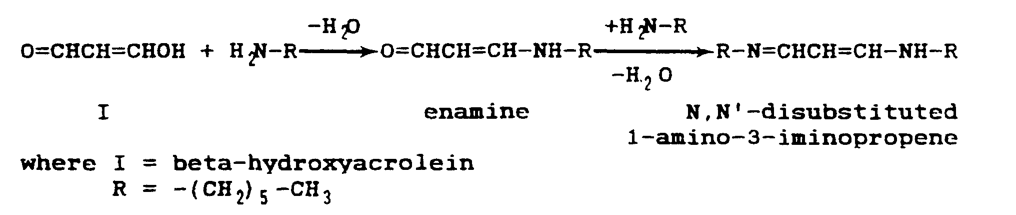
(d) The direct chemical addition of amines to 5-methyl-2-furfural has been described (Holdren and Hixon, 1946). A wide variety of aliphatic and aromatic primary amines can add to furfural in this manner, yielding Shiff base products (Dunlop and Peters, 1953, pg. 353). 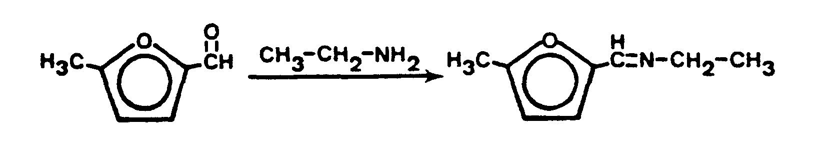
(e) As described by Dunlop and Peters (1953, pg. 373) earlier work demonstrated the ability of furfural to react with amino-sulfonic salts to produce furfurylideneaminosulfonates. 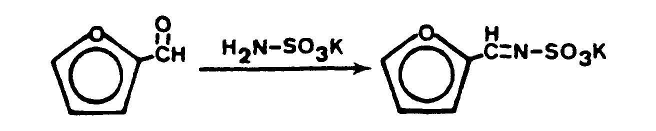
(f) The reaction of phenylaminoguanidine with furfural (Dunlop and Peters, 1953, pg. 371) may serve as an example of covalent furanaldehyde trapping with a hydrazine. 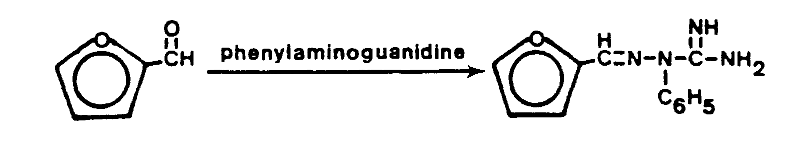
   It is proposed that the small molecular weight, absorbable, primary amine drugs and amine-related drugs described herein will have analogous behavior in vivo, as well as an additional characteristic which will facilitate disposal as urine metabolites. All of these drugs contain a carboxylic acid group to facilitate uptake and processing by the kidneys.

### (ii) Absorbable Drug Primary Agents

For any carboxylic acid primary agent listed herein as useful in the present invention, it is believed that the salt forms, free acid form, ester derivatives, amide derivatives and analogous non-aromatic benzene ring derivative (that is, cyclohexane carboxylic acid derivative) thereof will also be useful. The class of primary agents (molecular weight range 100 to 1,400) of the present invention may be summarized as noted below in chemical structures I, II and III. 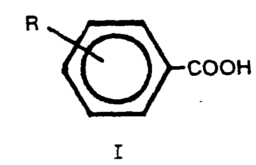
- R =: -NH₂
-aminoalkyl group having 1-10 carbons including hydrocarbon isomers and/or hydroxylated derivatives thereof
-NHC(=NH)NH₂
-(CH₂)ₙNHC(=NH)NH₂ where n = 1-10
-C(=NH)-NH₂
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
-(CH₂)ₙ-NHNHC(=NH)NH₂ where n = 1-10
-(CH₂)ₙ-CH=N-NHC(=NH)NH₂ where n = 1-10 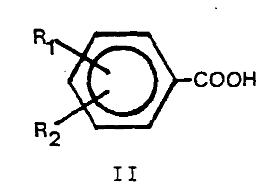
- R₁ =: -NH₂
-aminoalkyl group (1-10 carbons) including hydrocarbon isomers and/or hydroxylated derivatives thereof
-(CH₂)ₙNHC(=NH)NH₂ where n = 1-10
-C(=NH)-NH₂
-(CH₂)ₙ-CH=NC(=NH)NH₂ where n = 1-10
-NHC(=NH)NHNH₂ (continued) (CH₂)ₙNHC (=NH)NHNH, where n = 1-10
-(CH₂)ₙ-CH=NC ( =NH) NHNH where n = 1- 10
-NHNHC( =NH)NH₂
-(CH₂)ₙ-NHNHC(-NH)NH₂ where n = 1-10
.. (CH₂)ₙ-CH=N-NHC(=NH)NH₂
where n = 1-10
- R₂ =: -NH₂
-OH
-O-CH₃
-O-R^{'} with alkyloxy group R' having 2-10 carbons including hydrocarbon isomers and/or hydroxylated derivatives thereof
-aminoalkyl group (1-10 carbons) including hydrocarbon isomers and/or hydroxylated derivatives thereof
-SO₃H
-CH₃
-(CH₂)ₙCH₃ where n = 1-10 including hydrocarbon isomers and/or hydroxylated derivatives thereof 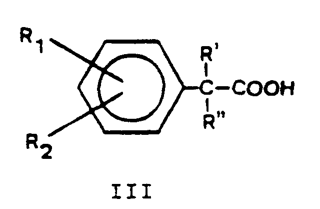
- R₁ =: -(CH₂)ₙ-NH₂ where n = 0-10 including isomers of the aminoalkyl group and hydroxylated derivatives thereof
-C(=NH)-NH₂
-NHC(=NH)NH₂
-(CH₂)ₙNHC(=NH)NH₂
where n = 1-10
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
-(CH₂)ₙ-NHNHC(=NH)NH₂ where n = 1-10
-(CH₂)ₙ-CH=N-NHC(=NH)NH- where n = 1-10 (continued)
- R₂ =: -NH₂
-H
-OH
-O-CH₃
-O-R₃ where alkyloxy group R₃ has 2-10 carbons including hydrocarbon isomers and/or hydroxylated derivatives thereof
-aminoalkyl group (1-10 carbons) including hydrocarbon isomers and/or hydroxylated derivatives thereof
-SO₃H
-CH₃
-(CH₂)ₙCH₃ where n = 1-10 including hydrocarbon isomers and/or hydroxylated derivatives thereof
- R' =: -H
-CH₃
-OH
- R" =: -H
-CH₃
-OH

### (iii) Mechanism of Action of Non-Absorbable Primary Amine and Aminc-Related Agents

The presence of aldehydes and ketones in the human diet may be a factor which may put the patient suffering from a chronic inflammatory disease further at risk. This might be especially important for victims of ileitis and colitis, as the damaging effects of inflammation site carbonyl compounds may be accentuated by direct exposure to dietary carbonyl agents. 5-Methyl furfural has been identified in the oil of roasted coffee and in oil of cloves (Dunlop and Peters, 1953, pg. 403). 5-Hydroxymethyl furfural has been found in sherry, port and brandy alcoholic beverages: honey and other sugar syrup products (Lever and coworkers, 1985). Levels of furfural (that is, 2-furanaldehyde or 2-furancarboxaldehyde) and 5-hydroxymethyl-2-furanaldehyde (that is, 5-hydroxymethyl furfural) as high as 4.5 mg/L and 93.2 mg/L, respectively, have been found in wine products (Shimizu and Watanabe, 1979). Furfural has also been detected in beer and distilled liquors (Dunlop and Peters, 1953, pg. 308), as well as in natural oil products such as oil of lime (Dunlop and Peters, 1953, pg. 280). Summarizing earlier work, Rice (1972) noted:
Small quantities of furfural occur in many foodstuffs, including - among many others - bread, coffee, processed fruits and fruit juices, and alcoholic beverages. In fact, whenever plant or animal tissue containing pentoses or hexoses is subjected to heat, the possibility arises that furfural, 5-hydroxymethyl furfural, and probably other furans as well will be produced.

Pettersen and Jellum (1972) referred to earlier work which demonstrated the generation of 2-furanaldehyde, 5-hydroxymethyl-2-furanaldehyde and 2,5-furandicarboxaldehyde during bread baking. In his food chemistry study, Baltes (1985) noted the presence of furfural in curing smoke tar; and the presence of furfural, 5-methyl-2-furfural, dihydrofuranone, 5-hydroxymethyl-2-furfural and 2,5-furandialdehyde in caramels. Baltes also examined the products obtained by Maillard reaction of glucose and phenylalanine and identified furfural and 2,5-di-(hydroxymethyl)-furan among the main components. Thus various furan aldehyde compounds have been identified in the human diet.

In addition, a wide variety of naturally occurring non-aromatic and aromatic aldehydes and ketones have been found in fruits and vegatables (Schauenstein and Esterbauer, 1977, pgs. 181-194). These include alkanals, alk-2,4-dienals, alk-2-enals, alk-1-en-3-ones, alpha-dicarbonyl compounds, beta-dicarbonyl compounds alkan-2-ones. Schauenstein and Estabauer have noted, in part, that:
Aliphatic carbonyl compounds represent the most important group of flavouring compounds in our foodstuffs. One finds them in all flavour extracts. They are either entirely, or in large measure, responsible for nearly all known flavours and determine, even when present in small amounts, the taste and odour of our foodstuffs, and beverages such as tea and coffee...(pg. 189)

As the presence of carbonyl agents in the diet is not restricted to fruits and vegatables, Schauenstein and Estabauer have further noted that:
Unsaturated aldehydes also arise through thermal degradation of carbohydrates, amino acids, and fats. Such thermal degradative processes are probably responsible for the presence of these aldehydes in boiled, fried, and baked foods. Unsaturated aldehydes have been detected in a large number of foodstuffs, such as potatoes, potato chips, poultry, meat, fish, salad oils, bread, and bakery products ...(pgs. 193-194)

As such, it is apparent that the diet is a significant source of carbonyl agents, and their presence may be a contributing factor in the etiology of chronic inflammatory diseases. Toxic properties of aliphatic aldehydes are well known, and toxic properties of furanaldehyde derivatives have been demonstrated in both in vivo and in vitro studies (Konecki and coworkers, 1974; Ulbricht and coworkers, 1984).

It is proposed that non-absorbable dietary supplements such as those defined below can be of health benefit by virtue of their ability to covalently trap dietary aldehydes and ketones. The agents described in this section can accomplish this function because they bear primary amine groups or derivatives thereof. As large molecular weight molecules which are non-digestible they have the capacity to pass through the digestive tract, acting in effect as another form of dietary fiber. These non-absorbable substances may be divided into three classes: (1) naturally occurring polyamine polysaccharides, (2) chemical derivatives of naturally occurring polysaccharides, and (3) synthetic polyamine polymers.

The fate of malondialdehyde given orally to rats may serve as an example of the metabolism of dietary aldehydes, and how an understanding of this process can be used to define non-absorbable carbonyl-trapping drugs. Studies by Draper and coworkers (1986) demonstrated that the primary form of "bound" MDA in rat or human urine is N-alpha-acetyl-epsilon-(2-propenal)lysine. This is the biologically alpha-N acetylated derivative of the MDA-lysine adduct N-epsilon-(2-propenal)lysine, as shown below.

Draper and coworkers (1986) were able to generate N-epsilon-(2-propenal)lysine in vitro by exposing beef muscle protein to MDA, followed by treatment with pepsin and hog intestinal juice. This indicates that the epsilon-amino groups of dietary protein 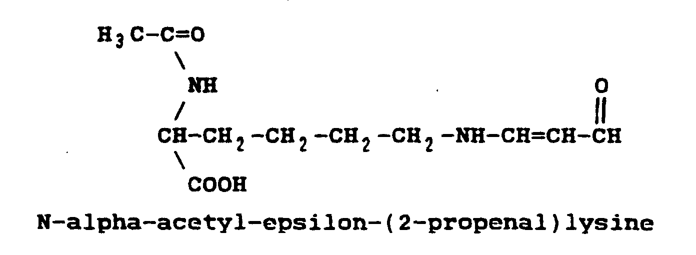 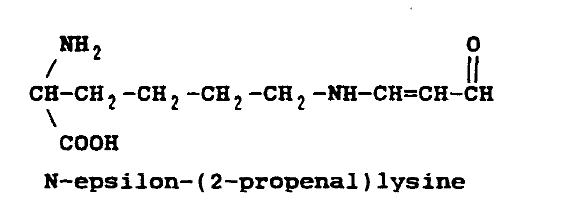 lysine residues can covalently bind dietary aldehyde under conditions found in the intestinal tract. As such, chemically analogous primary amine groups on non-absorbable drugs should also be capable of covalently binding dietary aldehydes under conditions to be found in the intestinal tract. In this case, however, the bound carbonyl species would be excreted in the faces, thus preventing subsequent in vivo exposure to dietary carbonyl agents.

In their study Draper and coworkers noted that N-alpha-acetyl-epsilon-(2-propenal)lysine was found in urine of fasted rats or animals fed on MDA-free diets, indicating that the MDA-lysine adduct also forms in vivo. These investigators referred to earlier work which demonstrated that the MDA concentration normally found in food is in the range of <0.1 to 10 ppm (0.1 to 10 µM), which gives some idea of dietary aldehyde concentrations.

### (iv) Examples of Non-Absorbable Drug Products Useful in the Present Invention

### (a). Naturally Occurring Amine-Containing Polysaccharides

Any naturally occurring polysaccharide featuring beta-1,2, beta-1,3, beta-1,4 and/or beta-1,6 linkages which contains aminosugars may be regarded as a non-digestible, potentially active carbonyl trapping agent. The chitin class of biopolymers may be cited as an example of such an agent, having the general structure of **poly-beta-(1→4)-N-acetyl-D-glucosamine.** A form of microcrystalline chitin has been described in which some of the acetyl groups have been removed, revealing free amine groups (Austin and coworkers, 1981, pg. 750). Chitins obtained from different sources feature different degrees of amine deacetylation (Austin and coworkers, 1981, pg. 752).

### (b). Chemical Derivatives of Naturally Occurring Polysaccharides

Various pretreatment procedures may be applied to naturally occurring polysaccharides prior to generation of chemical derivatives. Generation of microcrystalline polysaccharides is one example of such a pretreatment procedure. As applied to cellulose or chitin (Yalpani, 1988, pg. 389), this yields a colloidal processed form of polysaccharide featuring high porosity and enhanced susceptibility to chemical reactions. Generation of "microfibrillated" cellulose or chitin is another example of a pretreatment procedure which produces enhanced surface area, increased water retention capacity and enhanced chemical accessibility (Yalpani, 1988, pg. 390). Use of strong (> 18%) sodium hydroxide is still another recognized pretreatment, or activation, procedure found to be helpful as a starting point for preparing chemical derivatives of polysaccharides (Yalpani, 1988, pg. 214).

### (b)(1). Deacetylation of Naturally Occurring Polysaccharides

A variety of polysaccharides have been identified which are rich in N-acetylated residues. Upon chemical deacetylation these carbohydrates yield high molecular weight derivatives bearing primary amine groups directly linked to sugar carbons, that is, no sidearm spacer units present.
(i) Chitosan. This is the deacylated form of chitin. As described in the Merck Index (Budavari and coworkers, 1989, pg. 316) chitin is a cellulose-like biopolymer the composition of which consists mostly of N-acetyl-D-glucosamine residues co-valvently linked by beta-1,4 bonds. Chemical deacylation removes acetate, generating primary amine groups still covalently bound to the polysaccharide. Chitosan has recognized uses in water treatment, in photographic emulsions, and in improving the dyability of synthetic fabrics and fibers. The free amine groups in this substance also give it chelating properties (Austin and coworkers, 1981).
(ii) Chondroitin sulfate. This is a mucopolysaccharide found commonly in mammalian tissue. It consists of repeating disaccharide units, each of which has a D-glucuronic acid residue beta-1,4 linked to an N-acetylchondrosine residue (Merck Index, Budavari and coworkers, 1989, pg. 344).
(iii) Hyaluronic acid. This mucopolysaccharide is also found commonly in mammalian tissues. It consists of glucuronic acid and glucosamine residues bound by beta-1,3 and beta-1,4 linkages (Merck Index, Budavari and coworkers, 1989, pp. 751-752).
(iv) Keratan sulfate. This mammalian glycosaminoglycan consists of a repeating disaccharide unit of a C-6 sulfated C-2 N-acetylated sugar residue and a galactose residue linked by beta-1,4 bonds (Yalpani, 1988, pp. 27-28).

### (b)(2). Chemical Amination of Polysaccharides

(i) 2-Amino-2-deoxy-cellulose. Cellulose can be aminated by a process of selective oxidation, oximation and subsequent reduction with lithium aluminum hydride (Yalpani, 1988, pp. 281-282).
(ii) Alternative amination procedures. Aminodeoxy polysaccharides can also be prepared via azide or hydrazide intermediates or by reductive amination using sodium cyanoborohydride (Yalpani, 1988, pg. 281). Besides being applied to cellulose, other non-digestible polysaccharides such as curdlan (Yalpani, 1988, pg. 22) may be aminated by such chemical procedures.
(iii) 3-Aminopropylcellulose. Reaction of cyanoethylcellulose with borane-tetrahydrofuran or borane-dimethyl sulfide complexes in tetrahydrofuran generates 3-aminopropylcellulose (Yalpani, 1988, pgs. 250 and 255). In this derivative each primary amine group is at the end of a three carbon sidearm.
(iv) Aminoethylcellulose. This chemical has been previously marketed as an anion exchange column chromatography resin (Sigma Chemical Co. catalog, Feb. 1981) and used as such in protein purification studies (Fasold, 1975, pp 481-482).
(v) Other aminoalkyl-, amino(hydroxyalkyl)-, aminoalkyl-ether-, and amino(hydroxyalkyl)-ether- derivatives of cellulose, chitin and other naturally occurring non-digestible carbohydrates. Noting that the chemical methodology for producing such derivatives is documented in public domain literature, the biomedical application of such derivatives for therapeutic purposes described herein is also claimed. This would include:
   aminoalkyl derivatives of the formula
      **H**_{**2**}**N-(CH**_{**2**}**)**_{**n**}**-[carbohydrate]** where n = 1 - 10, including alkyl isomers;
   amino(hydroxyalkyl)- derivatives derivatives of the formula
      **H**_{**2**}**N-(CH**_{**2**}**)**_{**m**}**-CHOH-(CH**_{**2**}**)**_{**n**}**-[carbohydrate]**, where m = 0 - 10
      n = 0 - 10;
   aminoalkyl-ether- derivatives of the formula
      **H**_{**2**}**N-(CH**_{**2**}**)**_{**n**}**-O-[carbohydrate]**, where n = 1 - 10; and
   amino(hydroxyaklyl)-ether- derivatives of the formula
      **H**_{**2**}**N-(CH**_{**2**}**)**_{**m**}**-CHOH-(CH**_{**2**}**)**_{**n**}**-O-[carbohydrate]**, where m = 0 - 10
      n = 0 - 10
(vi) Aminobenzyl- derivatives of cellulose, chitin or other naturally occurring non-digestible carbohydrates. As the aromatic amine group is a weaker base than its aliphatic counterpart, this class of non-absorbable amines should be less chemically active than amino- and aminoalkyl- derivatives described above. These derivatives are of the following general structures:
   **H**_{**2**}**N-C**_{**6**}**H**_{**4**}**-(CH**_{**2**}**)**_{**n**}**-[carbohydrate] ;**
   **H**_{**2**}**N-CH**_{**2**}**-C**_{**6**}**H**_{**4**}**-(CH**_{**2**}**)**_{**n**}**-[carbohydrate];**
   **H**_{**2**}**N-C**_{**6**}**H**_{**4**}**-(CH**_{**2**}**)**_{**n**}**-O-[carbohydrate]** where n = 0 - 10; and
   **H**_{**2**}**N-C**_{**6**}**H**_{**4**}**-(CH**_{**2**}**)**_{**m**}**-CHOH-(CH**_{**2**}**)**_{**n**}**-O-[carbohydrate]** where m = 0 - 10
      n = 0 - 10
      This includes p-, o- and m-benzene ring amino- and aminomethylisomers, and alkyl group isomers.
(vii) guanidine and aminoguanidine derivatives of cellulose, chitin or other naturally occurring non-absorbable carbohydrates selected from the group consisting of:
   **H**_{**2**}**N-C(=NH)-[carbohydrate];**
   **H**_{**2**}**N-C(=NH)-(CH**_{**2**}**)**_{**n**}**-[carbohydrate]**, where n = 1-10, including hydrocarbon isomers and hydroxylated derivatives thereof;
   **H**_{**2**}**N-C(=NH)-O-(CH**_{**2**}**)**_{**n**}**-[carbohydrate]**, where n = 1-10, including hydrocarbon isomers, ether linkage isomers and hydroxylated derivatives thereof;
   **H**_{**2**}**N-C(=NH)-NH-[carbohydrate]**;
   **H**_{**2**}**N-C(=NH)-NH-(CH**_{**2**}**)**_{**n**}**-[carbohydrate]**, where n = 1-10, including hydrocarbon isomers and hydroxylated derivatives thereof;
   **H**_{**2**}**N-C(=NH)-NH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-C(=NH)-N=CH-(CH**_{**2**}**)**_{**n**}**-[carbohydrate]**, where n = 1-10, including hydrocarbon isomers and hydroxylated derivatives thereof;
   **H**_{**2**}**N-C(=NH)-N=CH-(CH**_{**2**}**)**_{**n**}**-O-[carbohydrate]**, where n = 1-10 , including hydrocarbon isomers and hydroxylated derivatives thereof;
   **H**_{**2**}**N-NHC(=NH)-NH-[carbohydrate];**
   **H**_{**2**}**N-NHC(=NH)-NH-(CH**_{**2**}**)**_{**n**}**-[carbohydrate]**, where n = 1-10, including hydrocarbon isomers and hydroxylated derivatives thereof;
   **H**_{**2**}**N-NHC(=NH)-NH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-NHC(=NH)-N=CH-(CH**_{**2**}**)**_{**n**}**-[carbohydrate]**, where n = 1-10, including hydrocarbon isomers and hydroxylated derivatives thereof;
   **H**_{**2**}**N-NHC(=NH)-N=CH-(CH**_{**2**}**)**_{**n**}**-O-[carbohydrate]**, where n = 1-10, including hydrocarbon isomers, ether linkage isomers and hydroxylated derivatives thereof;
   **H**_{**2**}**N-C(=NH)-NH-NH-[carbohydrate];**
   **H**_{**2**}**N-C(=NH)-NH-NH-(CH**_{**2**}**)**_{**n**}**-[carbohydrate],** where n = 1-10, including hydrocarbon isomers and hydroxylated derivatives thereof ;
   **H**_{**2**}**N-C(=NH)-NH-NH-(CH**_{**2**}**)**_{**n**}**-O-[carbohydrate],** where n = 1-10, including hydrocarbon isomers, ether linkage isomers and hydroxylated derivatives thereof;
   **H**_{**2**}**N-C(=NH)-NH-N=CH-(CH**_{**2**}**)**_{**n**}**-[carbohydrate],** where n = 1-10, including hydrocarbon isomers and hydroxylated derivatives thereof; and
   **H**_{**2**}**N-C(=NH)-NH-N=CH-(CH**_{**2**}**)**_{**n**}**-O-[carbohydrate],** where n = 1-10, including hydrocarbon isomers, ether linkage isomers and hydroxylated derivatives thereof

### (b)(3). Aminated Sucrose Polyesters

Mixtures of fatty acid hexa-, hepta- and octaesters of sucrose, known as sucrose polyester, are not hydrolyzed by pancreatic lipase enzymes and are not absorbed in the intestine (Jandacek, 1984). It is proposed and claimed herein that primary amine, aminoguanidine and guanidine derivatives of sucrose polyesters may be of benefit in reduction of dietary carbonyl substances, analogous to the proposed action of other non-absorbable agents described herein. Such derivatives of sucrose polyesters would include structures in which the carbonyl trapping functional group is in the omega-, omega-1 or other isomeric position(s) within the fatty acyl chains, fatty acyl chains having more than one nitrogen functional group and fatty acyl chains having hydroxyl groups. Such aminated sucrose polyesters may be used in pure form as a dietary supplement, or may be prepared as a coating on a particulate carrier such as cellulose or styrene divinylbenzene copolymer resin.

### (c). Synthetic Polyamine Polymers

(c)(1). Synthetic polysaccharides consisting partly or entirely of aminosugars bound by beta-1,2, beta-1,3, beta-1,4 and/or beta-1,6 linkages may be regarded as non-absorbable potential carbonyl trapping agents.

(c)(2). Mixed polysaccharide polymeric derivatives. Primary amine, aminoalkyl (one to ten carbons per alkyl group), amino-hydroxyalkyl (one to ten carbons per alkyl group and one to ten hydroxyl groups per alkyl group), aminoguanidine, aminoguanidinyl-alkyl (one to ten carbons per alkyl group), aminoalkyl-guanidinyl (one to ten carbons per alkyl group), guanidine, aminobenzene and aminoalkylbenzene (one to ten carbons per alkyl group) functional groups may be covalently attached to matrices such as epi-chlorohydrin copolymers of cellulose or chitin. Functional group spacer groups may include alkene as well as alkyl groups.

(c)(3). Non-polysaccharide polymeric derivatives. Primary amine, aminoalkyl (one to ten carbons per alkyl group), amino-hydroxyalkyl (one to ten carbons per alkyl group and one to ten hydroxyl groups per alkyl group), aminoguanidine, aminoguanidinylalkyl (one to ten carbons per alkyl group), aminoalkyl-guanidinyl (one to ten carbons per alkyl group), guanidine, aminobenzene and aminoalkylbenzene (one to ten carbons per alkyl group) functional groups may be covalently attached to a wide variety of synthetic non-digestible polymers. Functional group spacer groups may include alkene as well as alkyl groups. Like their sugar-based counterparts, these agents should be capable of reacting with dietary carbonyl compounds. Nitrogen-containing functional groups may be covalently attached to synthetic supports such as polystyrene, styrene-divinylbenzene copolymer, polyvinyl alcohol and crosslinked derivatives thereof.

### (v) Co-Administration of Antioxidants and Lipid Peroxidation Inhibitors

It is claimed that the therapeutic value of the primary agents described herein may be maximized by administration in conjunction with recognized antioxidant free radical trapping compounds such as vitamin E (Stuckey, 1968, pp. 214-215) or other agents previously recognized as adjunts which facilitate in vivo capability to inhibit lipid peroxidation, such as selenium (Stuckey, 1968, pg. 236). Citric acid may also be included in this category of co-administered agents, as it is recognized as having antioxidant properties (Budavari and coworkers, 1989, pg. 363). This agent is also recognized as an inhibitor of Maillard reactions (Stuckey, 1968, pg. 210). In a published list of agents which function to supplement the chain-breaking antioxidant property of vitamin E, Tappel (1970, pg. 1138) mentioned ubiquinol, seleno-amino acids and sulfhydryl compounds (such as, glutathione, sulfhydryl proteins and methionine). Butylated-hydroxytoluene (Frankel, 1987, pg. 81) and beta-carotene (Frankel, 1987, pg. 82) should also be included in this group.

### (vi) Prophylactic Vitamin Co-Administration

It is yet still another object of this invention that the safety and effectiveness of the products described herein may be optimized by co-administration of vitamins and derivatives thereof which may be inadvertently depleted by the therapeutic treatment of this invention or by the etiology of the disease being addressed, such as vitamin A (retinol), vitamin A aldehyde (retinal), vitamin A acid (retinoic acid), vitamin B₁ (thiamine), vitamin B₂ (riboflavin), vitamin B₆ (pyridoxine), vitamin B₁₂ (cyanocobalamin), vitamin D₂, vitamin D₃, vitamin H (biotin), vitamin K₁, vitamin K₁ oxide, vitamin(s) K₂, vitamin K₅ and vitamin K₆, vitamin K₇, vitamin K-S(II), vitamin L₁, vitamin L₂, vitamin U, alpha-carotene, beta-carotene, gamma-carotene, omega-carotene, carnitine (vitamin B_{T}), folic acid (vitamin Bc), folinic acid, niacinamide, nicotinic acid, pantothenic acid, pyridoxal, pyridoxal 5-phosphate, and pyridoxamine.

### (vii) Co-Administration of Metabolites at Risk of Depletion

It is another object of this invention that the safety and effectiveness of the products described herein may be optimized by co-administration of other metabolites, such as glycine, which may be depleted within the body during long term drug use. As many of the absorbable amine drugs described herein are excreted from the body as glycine conjugates, co-administration of glycine may be advisable. Coenzyme A is a required cofactor for hippuricase, the liver enzyme which adds glycine to benzoic acid derivatives. Activity of hippuricase in glycinating some of the absorbable carbonyl-trapping drugs described herein may sequester a disproportionate fraction of the endogenous coenzyme A pool. Hence co-administration of pantothenic acid, a metabolic precursor of coenzyme A, may also serve to optimize the therapeutic procedures described herein.

### (viii) Co-Administration of Sulfhydryl Agents

Noting the well documented ability of carbonyl agents to react with sulfhydryl groups (Jellum and coworkers, 1973), it is a further object of this invention that methionine and homo-cysteine may also be of clinical benefit as absorbable drugs capable of covalently binding aldehyde or ketone agents. It is also claimed that these drugs can be used most effectively when administered in conjunction with absorbable and non-absorbable amine and amine-related drugs described herein. Homocysteine contains a free sulfhydryl group. Methionine is converted in vivo to homocysteine by several enzymatic reactions which remove a methyl group. Thioctic acid, also known as alpha-lipoic acid, is also included in this category, as its structure includes a disulfide group. This agent, a recognized growth factor (Budavari and coworkers, 1989, pg. 1469), may tend to be depleted in the tissues of patients having chronic inflammatory diseases involving etiologies which include dysfunction of aldehyde and/or ketone metabolism. The ability of acetaldehyde to combine with thioctic acid, thus deactivating it, has been reported (Smith, 1976, pg. 195). Hence the inclusion of thioctic acid in pharmaceutical preparations such as those described herein may be of clinical benefit.

### (ix) Factors Affecting Dialy Dosage Schedule

A daily protocol of amine and amine-related drug consumption, in combination with co-agents defined herein, may be defined such that drug products are administered in timed-release and/or color coded tablets or capsules, so as to facilitate patient compliance and maximize therapeutic value.

### (4) Therapeutic Utilization

Although their primary etiologies remain to be determined, prior art information indicates that the secondary etiological processes of degenerative diseases such as rheumatoid artritis depends, in part, on chronic inflammation. In addition, evidence of increased lipid peroxidation has been demonstrated in studies on muscular dystrophy and multiple sclerosis (Halliwell and Gutteridge, 1985, pg. 125).

Reference one: Pearson and Shaw (1982, pg. 299) described the following summary of an arthritis patient taking vitamin E and vitamin A:
The correct dose of antioxidants for effective arthritis therapy must be determined by experimentation. The effective dose may be quite high. For example, a friend of ours who is a well-known artist in his fifties developed arthritis in his hands. This man's hands became so painful and stiff he could no longer use his fingers to remove the caps from his tubes of paint. He tried vitamin E at increasing dose levels. It was not until he got up to 10,000 I.U. of E and 20,000 I.U. of A per day that he obtained relief from the pain and crippling stiffness. His hands are now flexible and can be used to draw without difficulty. But they remain so only as long as our friend takes 10,000 I.U. of E and 20,000 I.U. of A a day, not less (he's tried).

This dosage of vitamin E far exceeds presently accepted levels of daily usage, which are generally regarded as being in the range of 400 I.U. per day. This particular combination of vitamins E and A, both lipophilic, would not be expected to inhibit any of the free radical reactions taking place in aqueous microenvironments. Nor would it chemically bind and thus deactivate any reactive aldehydes generated by the inflammatory process, as such aldehydes are water soluble.

Reference Two: Patient L.S. has a history of arthritis dating back to a serious automobile accident in 1980. By January of 1991 she had serious arthritic involvement of the lumbar spine and chronic hip and knee joint pain on a continuous basis. She had difficulty raising herself from a chair, required the assistance of a cane for activities as simple as walking from her front door to her car, was no longer able to go up or down a flight of stairs, and required use of a prescription analgesic drug every two hours during the night to sleep. She had participated in a program at the Pain Clinic of the University of Miami Medical School and at doctor's advice had used prescription drugs which included *Clinoril* (R) and *Anaprox* (R), both non-steroidal anti-inflammatory agents. At the recommendation of this inventor, patient L.S. began taking 800 I.0. vitamin E, 1. gm of methionine and 1.1 gm PABA per day for two months. Subsequently, vitamin E and methionine usage remained the same and PABA usage was increased to 2.2 gm per day, with the protocol continued on an indefinite basis.

When previously examined by an orthopedics physician a diagnosis was established which included:
...Lumbar spine X-Rays in AP and lateral views show extensive degenerative arthritic changes at multiple levels of the lumbar spine ... severe arthritic changes lumbar spine. Bursitis left greater trochanter clinically...She will always have a problem related to her underlying degenerative disease involving her lower back...She is favoring her left leg...Her straight leg raising is limited on the left side...

Ten weeks after after initiating this inventor's PABA/vitamin E/ methionine protocol, patient L.S. reported that her arthitis-related pain was much decreased and her functional status much improved. By four months into use of this therapeutic protocol patient L.S. had stopped using her cane, had a walking gait which was much improved, had taken to raking leaves in the yard as a form of exercise, and no longer required nighttime analgesics to sleep. At twelve months on this protocol, patient L. S. reported climbing and descending a flight of stairs without difficulty, and her ability to climb stairs has continued to improve. When re-examined by her orthopedies physician seven months after beginning therapy, who was not informed of her use of the PABA/vitamin E/methionine protocol, the doctor noted, in part:
This patient is markedly better. She has normal straight leg raising. She has no significant leg pain. She walks well on her toes and walks well on her heels now without any evidence of motor weakness. There is no limp present.

Unaware of the patient's collaborative effort with this inventor, the orthopedies physician was unable to provide an explanation of the marked improvement in the clinical status of patient L.S. At her office visit patient L. S. noted that she had stopped taking *Anaprox*, which her orthopedies physician had prescribed seven months earlier.

This inventor recognizes the novel and original composition of primary amine and amine-related benzoic acid derivatives as primary agents to be used with known antioxidant free radical trapping or inhibiting co-agents and not including vitamin C beyond its United States recommended daily allowance as a type of composition likely to have increased, synergistic properties for the treatment of chronic inflammatory diseases. This inventive strategy and the compositions thereof for clinical treatment of these diseases has not been previously recognized.

PABA, many of the other amine and amine-related primary agents, as well as the antioxidant free radical trapping co-agents are chemicals which have been previously synthesized and described. Yet the present invention recognizes a new and novel combination of therapeutic properties, never recognized previously by people trained in this field, and the clinical applications thereof. This invention constitutes a significant and practical advancement of the clinical therapeutic technology available for treating chronic inflammatory diseases.

Without further elaboration the foregoing will so fully illustrate my invention that others may, by applying current or future knowledge, adapt the same for use under various conditions of service.

### References Cited

Ahnfelt-Ronne, I et al. "Clinical evidence supporting the radical scavenger mechanism of 5-aminosalicylic acid" Gastroenterology 98:1162-1169 (1990)

Austin, PR et al. "Chitin: New facets of research" Science 212:749-753 (1981)

Baltes, W "Application of pyrolytic methods in food chemistry" J. Anal. Appl. Pyrol. 8:533-545 (1985)

Benedetti, A et al. "Foot-edema induced by carbonyl compounds originating from the peroxidation of liver microsomal lipids" Biochem. Pharmacol. 29:121-124 (1980)

Bingham, S and Cummings, JH "The use of 4-aminobenzoic acid as a marker to validate the completeness of 24 h urine collections in man" Clin. Sci. 64:629-635 (1983)

Borne, RF et al. "Anti-inflammatory activity of para-substituted N-benzenesulfonyl derivatives of anthranilic acid" J. Pharm. Sci. 63:615-617 (1974)

Brooks, PM and Schwarzer, AC "Combination chemotherapy in rheumatoid arthritis" Ann. Rheum. Dis. 50:507-509 (1991)

Budavari, S, ed. Merck Index, 11th ed., (Rahway, NJ, Merck & Co., 1989)

Carden, MJ et al. "2,5-Hexanedione neuropathy is associated with the covalent crosslinking of neurofilament proteins" Neurochem. Pathol. 5:25-35 (1986)

Chio, KS and Tappel, AL "Synthesis and characterization of the fluorescent products derived from malonaldehyde and amino acids" Biochemistry 8:2821-2827 (1969)

Chojkier, M et al. "Stimulation of collagen gene expression by ascorbic acid in cultured human fibroblasts" J. Biol. Chem. 264:16957-16962 (1989)

Del Maestro, RF "An approach to free radicals in medicine and biology" Acta Physiol. Scand. Suppl. 492:153-168 (1980) Demopoulos, HB "Control of free radicals in biologic systems" Fed. Proc. 32(8):1903-1908 (1973)

Demopoulos, HB et al. "The free radical pathology and the micro-circulation in the major central nervous system disorders" Acta Physiol. Scand. Suppl. 492:91-119 (1980)

Deutsche Gold- und Silber-Scheidcanstalt vorm. Roessler "Analgesic, antiinflammatory, and antipyretic p-aminobenzoic acid anilides" Chem. Abstr. 76:427 [abstract 140260d] (1972)

Dowd, AL Physician's Desk Reference, 47th ed. (Montvale, NJ, Medical Economics Data, 1993)

Draper, HH et al. "The metabolism of malondialdehyde" Lipids 21:305-307 (1986)

Drug Information for the Health Care Professional, 8th ed. (Rockville, MD, United States Pharmacopcial Convention, 1988) pgs. 111-113

Dull, BJ et al. "5-Aminosalicylate: oxidation by activated leukocytes and protection of cultured cells from oxidative damage" Biochem. Pharmacol. 36:2467-2472 (1987)

Dunlop, AP and Peters, FN The Furans (New York, Reinhold Publishing, 1953)

Fasold, H "Chromatography of proteins" in Chromatography: A Laboratory Handbook of Chromatographic and Electrophoretic Methods, 3rd ed., Heftmann, E, ed. (New York, Van Nostrand Reinhold, 1975) pp. 466-526

Feeney, RE et al. "Carbonyl-amine reactions in protein chemistry" Adv. Protein Chem. 29:135-203 (1975)

Flood, JF et al. "Two-drug combinations of memory enhancers: effect of dose ratio upon potency and therapeutic window, in mice" Life Sci. 42:2145-2154 (1988)

Frankel, EN "Secondary products of lipid oxidation" Chem. Phys. Lipids 44:73-85 (1987)

Ghose, A et al. "Protection with combinations of hydroxytryptophan and some thiol compounds against whole-body gamma irradiation" Int. J. Radiat. Biol. 44:175-181 (1983)

Goldstein, M et al. "The antiparkinsonian activity of dopamine agonists and their interaction with central dopamine receptor subtypes" Adv. Neurol. 53:101-106 (1990)

Gougerot, MM and Hewitt, J ["Scleroderma in patches, combined action of para-aminobenzoic acid and of alpha-tocopherol"] Bull. Soc. Fr. Dermatol. Syph. 58:42-43 (1951)

Grootveld, M and Halliwell, B "2,3-Dihydroxybenzoic acid is a product of human aspirin metabolism" Biochem. Pharmacol. 37:271-280 (1988)

Gutteridge, JM and Wilkins, S "Copper-dependent hydroxyl radical damage to ascorbic acid. Formation of a thiobarbituric acid-reactive product" FEBS Lett. 137:327-330 (1982)

Hall, ED "Beneficial effects of the 21-aminosteroid U74006F in acute CNS trauma and hypovolemic shock" Acta Anaesth. Belg. 38:421-425 (1987)

Halliwell, B and Gutteridge, JM "The importance of free radicals and catalytic metal ions in human diseases" Molec. Aspects Med. 8:89-193 (1985)

Higson, FK et al. "Iron enhancement of ascorbate toxicity" Free Rad. Res. Comms. 5:107-115 (1988)

Holdren, RF and Hixon, RM "The reaction of 2-methylfuran with formaldehyde and substituted ammonium chlorides" J. Am. Chem. Soc. 68:1198-1200 (1946)

Howie, MB and Bourke, E "Metabolism of p-aminobenzoic acid in the perfused livers of chronically uraemic rats" Clin. Sci. 56:9-14 (1979)

Huff, BB, cd., Physicians' Desk Reference, 34th ed. (Oradell, NJ, Medical Economics Co., 1980)

Hunter, MI et al. "Lipid peroxidation products and antioxidant proteins in plasma and cerebrospinal fluid from multiple sclerosis patients" Neurochem. Res. 10:1645-1652 (1985) Jackson, MJ et al. "Techniques for studying free radical damage in muscular dystrophy" Med. Biol. 62:135-138 (1984)

Jandacek, RJ "Studies with sucrose polyester" Int. J. Obes. 8(suppl. 1):13-21 (1984)

Jellum, E et al. "The presence of furan derivatives in patients receiving fructose-containing solutions intravenously" Clin. Chim. Acta 47:191-201 (1973)

Kar, NC and Pearson, CM "Catalase, superoxide dismutase, glutathione reductase and thiobarbituric acid-reactive products in normal and dystrophic human muscle" Clin. Chim. Acta 94:277-280 (1979)

Katsnelson, BA et al. "Trends and perspectives of the biological prophylaxis of silicosis" Environ. Health Perspect. 82:311-321 (1989)

Konecki, J et al. "Histochemical changes in the small intestine in acute furfural poisoning" Folia Histochem. Cytochem. 12:59-66 (1974)

Kontos, HA et al. "Prostaglandins in physiological and in certain pathological responses of the cerebral circulation" Fed. Proc. 40:2326-2330 (1981)

Kostyuk, VA et al; ["Antioxidative activity of the anti-arthritic drugs"] Vopr. Med. Khim. 36(3):37-39 (1990)

Kurdin, AN ["Free radical lipid oxidation in the pathogenesis of myocardial infarction"] Kardiologia 18:115-118 (1978)

Kushner, I, ed. Understanding Arthritis (New York, Charles Scribner's Sons, 1984)

Lever, M et al. "Automated fluorimetric determination of furfurals" Anal. Biochem. 144:6-14 (1985)

Maksimov, OB and Rebachuk, NM ["Screening for the presence of antioxidants in plant extracts"] Rastit. Resur. 21(2):216-220 (1985)

Mel'nikova, TA and Ryzhova, AF "Effect of p-aminobenzoic acid on the course of experimental myocardial infarction" translated from Byulleten Eksperimental'noi Biologii i Meditsiny 63(4):61-64 (1967)

Nakken, KF "The action of X-rays on dilute solutions of p-aminobenzoic acid" Radiation Res. 21:446-461 (1964)

Nakken, KF and Pihl, A "The X-ray-induced damage to the p-aminobenzoic acid moiety of folic acid and some other p-aminobenzoic acid conjugates irradiated in solution" Radiation Res. 27:19-31 (1966)

Olney, JW et al. "L-Cysteine, a bicarbonate-sensitive endogenous excitotoxin" Science 248:596-599 (1990)

Ortwerth, BJ and Olesen, PR "Ascorbic acid-induced crosslinking of lens proteins: evidence supporting a Maillard reaction" Biochim. Biophys. Acta 956:10-22 (1988)

Passwater, RA The Antioxidants. The Nutrients that Guard the Body Against Cancer, Heart Discase, Arthritis and Allergies - and Even Slow the Aging Process (New Canaan, CT, Keats Publishing, 1985)

Pearson, D and Shaw, S Life Extension. A Practical Scientific Approach (New York, Warner Books, 1982)

Peppercorn, MA "Sulfasalazine: Pharmacology, clinbical use, toxicity, and related new drug development" Ann. Intern. Med. 101:377-386 (1984)

Pettersen, JE and Jellum, E "The identification and metabolic origin of 2-furoylglycine and 2,5-furandicarboxylic acid in human urine" Clin. Chim. Acta 41:199-207 (1972)

Priestley, GC and Brown, JC "Effects of potassium para-aminobenzoate on growth and macromolecule synthesis in fibroblasts cultured from normal and sclerodermatous human skin, and rheumatoid synovial cells" J. Invest. Dermatol. 72:161-164 (1979)

Pryor, WA et al. "Autoxidation of polyunsaturated fatty acids. Part 1. Effect of ozone on the autoxidation of neat methyl linoleate and methyl linolenate" Arch. Environ. Health 31:201-210 (1976)

Repine, JE et al. "Generation of hydroxyl radical by enzymes, chemicals, and human phagocytes in vitro. Detection with the anti-inflammatory agent, dimethyl sulfoxide" J. Clin. Invest. 64:1642-1651 (1979)

Rice, EW "Furfural: exogenous precursor of certain urinary furans and possible toxicologic agent in humans" Clin. Chem. 18:1550-1551 (1972)

Richmond, R et al. "Superoxide-dependent formation of hydroxyl radicals: detection of hydroxyl radicals by the hydroxylation of aromatic compounds" Anal.Biochem. 118:328-335 (1981)

Rinne, UK "New strategies in the treatment of early Parkinson's disease" Acta Neurol. Scand. 84(suppl. 136):95-98 (1991)

Rowley, D et al. "Lipid peroxidation in rheumatoid arthritis: thiobarbituric acid-reactive material and catalytic iron salts in synovial fluid from rheumatoid patients" Clin. Sci. 66:691-695 (1984)

Sawicki, E et al. "Comparison of spectrophotometric and spectrophotofluorometric methods for the determination of malonaldehyde" Anal. Chem. 35:199-205 (1963)

Schauenstein, E and Esterbauer, H Aldehydes in Biological Systems. Their Natural Occurrence and Biological Activities (London, Pion Limited, 1977)

Scott, CC and Robbins, EB "Toxicity of p-aminobenzoic acid" Proc. Soc. Exp. Biol. Med. 49:184-186 (1942)

Scott, G "Antioxidants: Radical chain-breaking mechanisms" in Atmospheric Oxidation and Antioxidants (New York, Elsevier Publishing Co., 1965)

Shimada, O and Yasuda, H "Lipid peroxidation and its inhibition by tinoridine. II. Ascorbic acid-induced lipid peroxidation of rat liver mitochondria" Biochim. Biophys, Acta 572:531-536 (1979)

Shimizu, J and Watanabe, M "Gas chromatographic analysis of furfural and hydroxymethyl-furfural in wine" Agric. Biol. Chem. 43:1365-1366 (1979)

Sigma Chemical Co. catalog, Feb. 1981 (Saint Louis)

Sigma Chemical Co. catalog, 1993 (Saint Louis)

Slight, SH et al. "Glycation of lens proteins by the oxidation products of ascorbic acid" Biochim. Biophys. Acta 1038:367-374 (1990)

Smith, WT "Nutritional deficiencies and disorders" in Greenfield's Neurology, Blackwood, W and Corsellis, JAN, eds. (Chicago, Year Book Medical Publishers, 1976) pp. 194-237

Stuckey, BN "Antioxidants as food stabilizers" in CRC Handbook of Food Additives, Furia, TE, ed. (West Palm Beach, FL, CRC Press, 1968)

Tappel, AL "Biological antioxidant protection against lipid peroxidation damage" Am. J. Clin. Nutr. 23:1137-1139 (1970)

Thiele, K "p-Aminobenzoic acid anilides" Chem. Abstr. 75:462 [abstract 35466g] (1971)

Toufexis, A "The new scoop on vitamins" Time 139(14):54-59 (1992)

Ulbricht, RJ et al. "A review of 5-hydroxymethylfurfural (HMF) in parenteral solutions" Exp. Appl. Toxicol. 4:843-853 (1984) Warner-Lambert Co., United States Patent No. 5,002,703, "Benzoic acid and benzoic acid ester derivatives having antiinflammatory and analgesic activity," issued 8/25/87

Weissmann, G "Aspirin" Scientific American 264:84-90 (1991)

Weizman, Z et al. "Bentiromide test for assessing pancreatic dysfunction using analysis of para-aminobenzoic acid in plasma and urine" Gastroenterology 89:596-604 (1985)

Winitz, M et al. "Studies in metabolic nutrition employing chemically defined diets. I. Extended feeding of normal human adult males" Am. J. Clin. Nutr. 23:525-545 (1970)

Wong, SF et al. "The role of superoxide and hydroxyl radicals in the degradation of hyaluronic acid induced by metal ions and by ascorbic acid" J. Inorg. Biochem. 14:127-134 (1981)

Yalpani, M Polysaccharides: Syntheses, Modifications and Structure-Property Relations (New York, Elsevier, 1988)

Young, DS et al. "Influence of a chemically defined diet on the composition of serum and urine" Clin. Chem. 17:765-773 (1971)

Zarafonetis, CD "Clinical use of para-aminobenzoic acid" Texas State J. Med. 49:666-672 (1953)

Zarafonetis, CJ "Antifibrotic therapy with Potaba" Am. J. Med. Sci. 248:550-561 (1964)

Zarafonetis, CJ et al. "Retrospective studies in scleroderma: effect of potassium para-aminobenzoate on survival" J. Clin. Epidemiol. 41:193-205 (1988)

Zivin, JA and Choi, DW "Stroke therapy" Sci. Am. 265:56-63 (1991)

Zuckerman, JE et al. "Evaluation of antifibrotic drugs in bleomycin-induced pulmonary fibrosis in hamsters" J. Pharmacol. Exp. Ther. 213:425-431 (1980)

## Claims

1. Use of a pharmaceutical composition for the manufacture of a medicament for the treatment of the symptoms of chronic inflammatory disorders featuring pathology based in part on increased lipid peroxidation, wherein a primary and co-agent combination produces an anti-inflammatory and analgesic effect, wherein said disorders are selected from chronic gingivitis, chronic periodontitis, chronic autoimmune gastritis, ileitis, colitis, interstitial cystitis, arthritis, tendinitis, carpal tunnel syndrome and other cumulative trauma disorders, systemic lupus erythematosus, autoimmune vasculitis, asbestosis, silicosis, chronic obstructive pulmonary disease, Lyme disease, inflammatory myopathies, status epilepticus, inflammatory neuropathies, myasthenia gravis, as well as lessening of inflammatory site edema, and treatment of post-event ischemia and reperfusion symptomology resulting from acute central nervous system trauma, stroke and myocardial infarction; excluding multiple sclerosis;
the composition comprising a water soluble, low molecular weight primary agent in the molecular weight range of 100 to 1400 and at least one co-agent;
the primary agent of said pharmaceutical composition being selected from:
the free acid forms, salts, benzene ring isomers, amide derivatives, carboxylic acid ester derivatives and analogous non-aromatic benzene ring derivatives of : 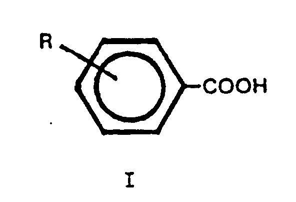
wherein R is selected from :
-NH₂
-aminoalkyl group having 1-10 carbons including hydrocarbon isomers and/or hydroxylated derivatives thereof
-NHC(=NH)NH₂
-(CH₂)ₙNHC(=NH)NH₂ where n = 1-10
-C(=NH)-NH₂
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
- (CH₂)ₙ-NHNHC(=NH)NH₂ where n = 1-10
-(CH₂)ₙ-CH=N-NHC(=NH)NH₂ where n = 1-10 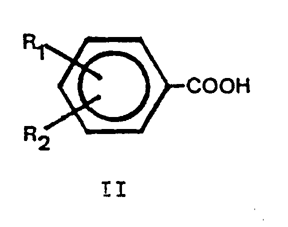 wherein R₁ is selected from :
-NH₂
-aminoalkyl group (1-10 carbons) including hydrocarbon isomers and/or hydroxylated derivatives thereof
-(CH₂)ₙNHC(=NH)NH₂ where n = 1-10
-C(=NH)-NH₂
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
-(CH₂)ₙ-NHNHC(=NH)NH₂ where n = 1-10
- (CH₂)ₙ-CH=N-NHC(=NH)NH₂ where n = 1-10
and wherein R₂ is selected from :
-NH₂
-OH
-O-CH₃
-O-R' with alkyloxy group R' having 2-10 carbons including hydrocarbon isomers and/or hydroxylated derivatives thereof
-aminoalkyl group (1-10 carbons) including hydrocarbon isomers and/or hydroxylated derivatives thereof
-SO₃H
-CH₃
-(CH₂)ₙCH₃ where n = 1-10 including hydrocarbon isomers and/or hydroxylated derivatives thereof 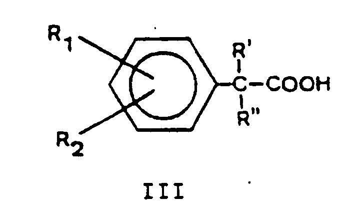 wherein R₁ is selected from :
-(CH₂)ₙ-NH₂ where n = 0-10 including isomers of the aminoalkyl group and hydroxylated derivatives thereof
-C(=NH)-NH₂
-NHC(=NH)NH₂
-(CH₂)ₙNHC(=NH)NH₂ where n = 1-10
-(CH₂)ₙ-CH=NC(=NH)NH₂ where n = 1-10
-NHC(=NH)NHNH₂
-(CH₂)ₙNHC(=NH)NHNH₂ where n = 1-10
-(CH₂)ₙ-CH=NC(=NH)NHNH₂ where n = 1-10
-NHNHC(=NH)NH₂
-(CH₂)ₙ-NHNHC(=NH)NH₂ where n = 1-10
-(CH₂)ₙ-CH=N-NHC(=NH)NH₂ where n = 1-10
wherein R₂ is selected from :
-NH₂
-H
-OH
-O-CH₃
-O-R₃ where alkyloxy group R₃ has 2-10 carbons including hydrocarbon isomers and/or hydroxylated derivatives thereof
-aminoalkyl group (1-10 carbons) including hydrocarbon isomers and/or hydroxylated derivatives thereof
-SO₃H
-CH₃
-(CH₂)ₙCH₃ where n = 1-10 including hydrocarbon isomers and/or hydroxylated derivatives thereof
and wherein R' and R" are independently selected from:
-H
-CH₃
-OH,
in association with a pharmaceutically acceptable carrier thereof, for administration of the said primary agent in a dosage range of from one gram/day to 20 grams/day,
**characterized in that**
said co-agent is selected from :
a. naturally occurring polysaccharides having beta-1,2, beta-1,3, beta-1,4 and/or beta-1,6 linkages containing aminosugars including the chitin class of biopolymers having the general structure of **poly-beta-(1->4)-N-acetyl-D-glucosamine,** and
bearing at least one free primary amine group;
b. deacetylated naturally occurring polysaccharides, having at least one N-acetylated residue, including chitosan, deacetylated chondroitin sulfate, or deacetylated hyaluronic acid;
c. chemically aminated polysaccharides selected from :
aminodeoxy polysaccharides such as 2-amino-2-deoxycellulose;
aminoalkyl-, amino(hydroxyalkyl)-, aminoalkyl-ether-, and
amino(hydroxyalkyl)-ether- derivatives of cellulose, chitin and other naturally occurring non-digestible carbohydrates selected from the group consisting of
**H**_{**2**}**N-(CH**_{**2**}**)**_{**n**}**-[carbohydrate]** where n = 1-10, including alkyl isomers;
**H**_{**2**}**N(CH**_{**2**}**)**_{**m**}**-CHOH-(CH**_{**2**}**)**_{**n**}**-[carbohydrate]**, where m = 0-10 and n = 0-10;
**H**_{**2**}**N(CH**_{**2**}**)**_{**n**}**-O-[carbohydrate]** where n = 1-10;
**H**_{**2**}**N-(CH**_{**2**}**)**_{**m**}**-CHOH(CH**_{**2**}**)**_{**n**}**-O-[carbohydrate]** where m = 0-10 and n = 0-10;
aminobenzyl- derivatives of cellulose, chitin or other naturally occurring non-digestible carbohydrates selected from
**H**_{**2**}**N-C**_{**6**}**H**_{**4**}**-(CH**_{**2**}**)**_{**n**}**-[carbohydrate],**
**H**_{**2**}**N-CH**_{**2**}**-C**_{**6**}**H**_{**4**}**-(CH**_{**2**}**)**_{**n**}**-[carbohydrate],**
**H**_{**2**}**N-C**_{**6**}**H**_{**4**}**-(CH**_{**2**}**)**_{**n**}**-O-[carbohydrate]** where n = 0 -10, and
**H**_{**2**}**N-C**_{**6**}**H**_{**4**}**-(CH**_{**2**}**)**_{**m**}**-CHOH-(CH**_{**2**}**)**_{**n**}**-O-[carbohydrate]** where m = 0-10
and n = 0-10, including p-, o- and m-benzene ring amino-isomers, aminomethyl- isomers and alkyl group isomers thereof;
guanidine and aminoguanidine derivatives of cellulose,
chitin or other naturally occurring non-absorbable carbohydrates selected from :
**H**_{**2**}**N-C(=NH)-[carbohydrate];**
**H**_{**2**}**N-C(=NH)-(CH**_{**2**}**)**_{**n**}**-[carbohydrate]**, where n = 1-10, including hydrocarbon isomers and hydroxylated derivatives thereof;
**H**_{**2**}**N-C(=NH)-O-(CH**_{**2**}**)**_{**n**}**-[carbohydrate]**, where n = 1-10, including hydrocarbon isomers, ether linkage isomers and hydroxylated derivatives thereof;
**H**_{**2**}**N-C(=NH)-NH-[carbohydrate];**
**H**_{**2**}**N-C(=NH)-NH-(CH**_{**2**}**)**_{**n**}**-[carbohydrate]**, where n = 1-10, including hydrocarbon isomers and hydroxylated derivatives thereof;
**H**_{**2**}**N-C(=NH)-NH-(CH**_{**2**}**)**_{**n**}**-O-[carbohydrate]**, where n = 1-10, including hydrocarbon isomers, ether linkage isomers and
hydroxylated derivatives thereof;
**H**_{**2**}**N-C(=NH)-N=CH(CH**_{**2**}**)**_{**n**}**-[carbohydrate**], where n = 1-10,
including hydrocarbon isomers and hydroxylated derivatives thereof;
**H**_{**2**}**N-C(=NH)-N=CH-(CH**_{**2**}**)**_{**n**}**-O-[carbohydrate]**, where n = 1-10, in-cluding hydrocarbon isomers and hydroxylated derivatives thereof;
**H**_{**2**}**N-NHC(=NH)-NH-[carbohydrate];**
**H**_{**2**}**N-NHC(=NH)-NH-(CH**_{**2**}**)**_{**n**}**-[carbohydrate**], where n = 1-10,
including hydrocarbon isomers and hydroxylated derivatives thereof;
**H**_{**2**}**N-NHC(=NH)-NH-(CH**_{**2**}**)**_{**n**}**-O-[carbohydrate]**, where n = 1-10, including hydrocarbon isomers, ether linkage isomers and hydroxylated derivatives thereof;
**H**_{**2**}**N-NHC(=NH)-N=CH(CH**_{**2**}**)**_{**n**}**-[carbohydrate]**, where n = 1-10, including hydrocarbon isomers and hydroxylated derivatives thereof;
**H**_{**2**}**N-NHC(=NH)-N=CH-(CH**_{**2**}**)**_{**n**}**-O-[carbohydrate]**, where n = 1-10, including hydrocarbon isomers, ether linkage isomers and hydroxylated derivatives thereof;
**H**_{**2**}**N-C(=NH)-NH-NH-[carbohydrate];**
**H**_{**2**}**N-C(=NH)-NH-NH-(CH**_{**2**}**)**_{**n**}**-[carbohydrate]**, where n = 1-10, including hydrocarbon isomers and hydroxylated derivatives thereof;
**H**_{**2**}**N-C(=NH)-NH-NH-(CH**_{**2**}**)**_{**n**}**-O-[carbohydrate]**, where n = 1-10, including hydrocarbon isomers, ether linkage isomers and
hydroxylated derivatives thereof;
**H**_{**2**}**N-C(=NH)-NH-N=CH-(CH**_{**2**}**)**_{**n**}**-[carbohydrate]**, where n = 1-10, including hydrocarbon isomers and hydroxylated derivatives thereof;
**H**_{**2**}**N-C(=NH)-NH-N=CH-(CH**_{**2**}**)**_{**n**}**-O-[carbohydrate]**, where n = 1-10, including hydrocarbon isomers, ether linkage isomers and hydroxylated derivatives thereof;
d. primary amine, aminoguanidine and guanidine derivatives of sucrose polyesters having one or more carbonyl trapping functional group per molecule wherein each carbonyl trapping functional group is in the omega-, omega-1 or other isomeric position within the fatty acyl chains, wherein each fatty acyl chain may have from 3 to 26 carbons, from one to five nitrogen functional groups and from one to 24 hydroxyl groups;
e. synthetic polysaccharides consisting partly or entirely of aminosugars bound by beta-1,2, beta-1,3, beta-1,4 and/or beta-1,6 linkages;
f. mixed polysaccharide polymeric derivatives wherein primary amine, aminoalkyl (one to ten carbons per alkyl group), amino-hydroxyalkyl (one to ten carbons per alkyl group and one to ten hydroxyl groups per alkyl group), aminoguanidine, aminoguanidinylalkyl (one to ten carbons per alkyl group), aminoalkyl-guanidinyl (one to ten carbons per alkyl group), guanidine, aminobenzene and/or aminoalkylbenzene (one to ten carbons per alkyl group) functional groups are covalently attached to matrices such as epichlorohydrin copolymers of cellulose or chitin and wherein hydrocarbon spacer groups may include alkene as well as alkyl groups; and
g. non-polysaccharide polymeric derivatives wherein primary amine, aminoalkyl (one to ten carbons per alkyl group), aminohydroxyalkyl (one to ten carbons per alkyl group and one to ten hydroxyl groups per alkyl group), aminoguanidine, aminoguanidinylalkyl (one to ten carbons per alkyl group), aminoalkylguanidinyl (one to ten carbons per alkyl group), guanidine, aminobenzene and/or aminoalkylbenzene (one to ten carbons per alkyl group) functional groups are covalently attached to a synthetic non-digestible polymer selected from polystyrene, styrene-divinylbenzene copolymer, polyvinyl alcohol and crosslinked derivatives thereof, and wherein hydrocarbon spacer groups may include alkene as well as alkyl groups; in a microfibrillated form or microcrystalline form having enhanced surface area, increased porosity, increased water retention capacity and enhanced chemical accessibility, for administration of said co-agent in a dosage range of from one gram/day to 40 grams/day.

2. The use according to claim 1, **characterized in that** said co-agent is in a microfibrillated form or microcrystalline form having enhanced surface area, increased porosity, increased water retention capacity and enhanced chemical accessibility.

3. The use of claim 1, **characterized in that** the manufactured medicament is useful for oral or intravenous administration of said co-agent.

4. The use according to claims 1 to 3, **characterized in that** the manufactured medicament is useful for administration of the primary agent in a dosage of from 10 mg/kg/day to 1.0 g/kg/day, or for administration of the non-absorbable polyamine substance in a dosage of from 10 mg/kg/day to 1.0 g/kg/day.

## Patentansprüche

1. Nutzung einer pharmazeutischen Zusammensetzung für die Herstellung eines Medikaments zur Behandlung von Symptomen von chronischen Entzündungskrankheiten deren Pathologie zum Teil auf einer erhöhten Peroxidierung von Fetten beruht, worin eine primäre und eine Ko-Agenz Kombination einen entzündungshemmenden und schmerzstillenden Effekt produziert, wobei die besagten Krankheiten die folgende Selektion umfasst: chronische Zahnfleischentzündung (Gingivitis und Periodontitis), chronische autoimmun Gastritis, Entzündung, Kolitis (Mastdarmentzündung), interdistielle Blasenentzündung od. ~ Zystitis, Arthritis, Tendinitis (Sehnenentzündung), Karpaltunnelsyndrom und andere kumulative Trauma Erkrankungen, systemischer Lupus erythematodes, Autoimmunvaskulitis, Asbestose, Silikose, chronisch obstruktive Lungenerkrankung, Borreliose, Myopathien, Status epilepticus, entzündliche Neuropathien, Myastenia gravis, wie auch Linderung von entzündlichen Ödemen, und Behandlung von Ischämie und Reperfusions Symptomologie resultierend von akutem Trauma des zentralen Nervensystems, Schlaganfall und Herzinfarkt; auschliesslich Multipler Sklerose;
die Zusammensetzung bestehend aus einem primären, wasserlöslichem Agenz von kleinem Molekulargewicht mit einem Molekulargewicht zwischen 100 und 1400 und mindestens einem Ko-Agenz;
das primäre Agenz von besagter pharmazeutischen Zusammensetzung bestehend aus:
den freien Säureformen, Salzen, Benzenring Isomeren, Amid Derivaten, Carboxyiicsäure-Ester Derivaten, und analogen nichtaromatischen Benzenring Derivaten von: 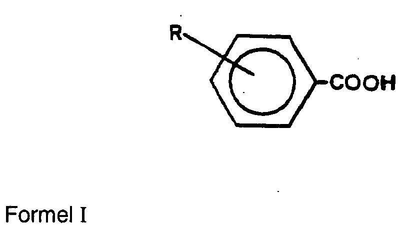
wobei R dargestellt ist bei:
-NH₂
-Aminoalkylgruppe mit 1-10 Kohlenstoffen, einschliesslich der Kohlenwasserstoff Isomere und/oder der hydroxylierten Derivate davon
- NHC(=NH)NH₂
-(CH₂)ₙNHC(=NH)NH₂, wobei n = 1-10 ist
-C(=NH)-NH₂
-(CH₂)ₙ-CH=NC(=NH)NH₂, wobei n = 1-10 ist
-NHC(=NH)NHNH₂, wobei n = 1-10 ist
-(CH₂)ₙNHC(=NH)NHNH₂
-(CH₂)ₙ-CH=NC(=NH)NHNH₂
-NHNHC(=NH)NH₂
-(CH₂)ₙ-NHNHC(=NH)NH₂, wobei n = 1-10 ist
-(CH₂)ₙ-CH=N-NHC(=NH)NH₂, wobei n = 1-10 ist 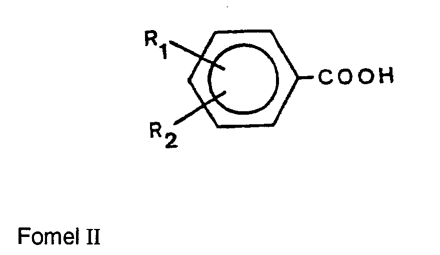
wobei R₁ dargestellt ist bei
-NH₂
- Aminoalkylgruppe (1-10 Kohlenstoffe), einschliesslich der Kohlenwasserstoff Isomere und/oder der hydroxylierten Derivaten davon
-(CH₂)ₙNHC(=NH)NH₂, wobei n = 1-10 ist
-C(=NH)-NH₂
-(CH₂)ₙ-CH=NC(=NH)NH₂, wobei n = 1-10 ist
-(CH₂)ₙ-CH=NC(=NH)NH₂, wobei n = 1-10 ist
-NHC(=NH)NHNH₂
-(CH₂)ₙ-NHC(=NH)NHNH₂, wobei n = 1-10 ist
-(CH₂)ₙ-CH=NC(=NH)NHNH₂, wobei n = 1-10 ist
-NHNHC(=NH)NH₂
-(CH₂)ₙ-NHNHC(=NH)NH₂, wobei n = 1-10 ist
-(CH₂)ₙ-CH=N-NHC(=NH)NH₂, wobei n = 1-10 ist
und wobei R₂ dargestellt ist bei:
-NH₂
-OH
-O-CH₃
-O-R' mit Alkyloxygruppe R' bestehen aus 2-10 Kohlenstoffen, einschliesslich der Kohlenwasserstoff Isomere und/oder der hydroxylierte Derivaten davon
- Aminoalkylgruppe (1-10 Kohlenstoffe), einschliesslich der Kohlenwasserstoff Isomere und/oder hydroxylierten Derivate davon
-SO₃H
-CH₃
-(CH₂)ₙCH₃, wobei n = 1-10 ist, einschliesslich der Kohlenwasserstoff Isomere und/oder der hydroxylierten Derivate davon 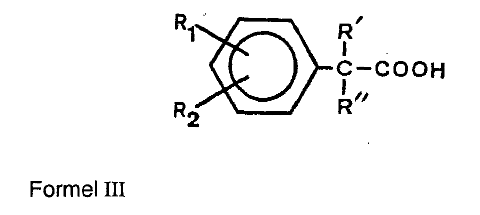 wobei R₁ dargestellt ist bei:
-(CH₂)ₙ-NH₂, wobei n = 1-10 ist, einschliesslich der Isomere der Aminoalkylgruppe und der hydroxylierten Derivate davon
-C(=NH)-NH₂
-NHC(=NH)-NH₂
-(CH₂)ₙNHC(=NH)-NH₂, wobei n = 1-10 ist
-(CH₂)ₙ-CH=NC(=NH)NH₂, wobei n = 1-10 ist
-NHC(=NH)NHNH₂
-(CH₂)ₙ-NHC(=NH)NHNH₂, wobei n = 1-10 ist
-(CH₂)ₙ-CH=NC(=NH)NHNH₂, wobei n = 1-10 ist
-NHNHC(=NH)NH₂
-(CH₂)ₙ-NHNHC(=NH)NH₂, wobei n = 1-10 ist wobei R₂ dargestellt ist bei:
-NH₂
-H
-OH
-O-CH₃
-O-R₃ wobei die Alkyloxy R₃ 2-10 Kohlenstoffe hat, einschliesslich der Kohlenwasserstoff Isomere und/oder der hydroxylierten Derivate davon
- Aminoalkylgruppe (1-10 Kohlenstoffe), einschliesslich der Kohlenwasserstoff Isomere und/oder der hydroxylierten Derivate davon
-SO₃H
-CH₃
-(CH₂)ₙCH₃, wobei n = 1-10 ist, einschliesslich der Kohlenwasserstoff Isomere und/oder der hydroxylierten Derivate davon
und wobei R' und R" unabhängig voneinander dargestellt sind als:
-H
-CH₃
-OH
in Verbindung mit einem pharmazeutisch akzeptablem Träger davon, zur Verabreichung der besagten primären Agenz in einem Dosisbereich zwischen einem Gramm/Tag bis zu 20 Gramm/Tag,
**charakterisiert darin dass**
besagtes Ko-Agenz bestehend aus:
a. einem natürlich vorkommenden Polysaccharid mit beta-1,2, beta-1,3, beta-1,4, und/oder beta-1,6 Bindungen, die Aminozucker enthalten, einschliesslich der Chitin Klasse von Biopolymeren mit der allgemeinen Struktur von **Polybeta-(1->4)-N-acetyl-D-glucosamin**, und
wenigstens eine freie primare Amingruppe tragen;
b. deacetylierte natürlich vorkommende Polysaccharide mit wenigstens einer N-acetylierten Restgruppe, einschliesslich Chitosan, deacetyliertem Chomdroitinsulfat, oder deacetylierter Hyalurinsäure;
c. chemisch aminierten Polysacchariden bestehend aus: Aminodeoxy-polysacchariden wie 2-Amino-2-Deoxy-Cellulose;
Aminoalkyl-, Amino(hydroxyalkyl)-, Aminoalky-äther-, und Amino(hydroxyalkyl)-äther Derivaten von Cellulose, Chitin, und anderen natürlich vorkommenden unverdaubaren Kohlenhydraten bestehend aus:
**H**_{**2**}**N-(CH**_{**2**}**)**_{**n**}**-[Kohlenhydrat]**, wobei n = 1-10 ist, einschliesslich der Alkylisomere;
**H**_{**2**}**N-(CH**_{**2**}**)**_{**m**}**-CHOH-(CH**_{**2**}**)**_{**n**}**-[Kohlenhydrat],** wobei m = 1-10 und n = 1-10 ist;
**H**_{**2**}**N-(CH**_{**2**}**)**_{**n**}**-O-[Kohlenhydrat],** wobei n = 1-10 ist;
**H**_{**2**}**N-(CH**_{**2**}**)**_{**m**}**-CHOH-(CH**_{**2**}**)**_{**n**}**-O-[Kohlenhydrat],** wobei m = 1-10 und n = 1-10 ist;
Amlnobenzyl-Derivaten von Cellulose, Chitin, oder anderen natürlich vorkommenden unverdaubaren Kohlenhydraten bestehend aus:
**H**_{**2**}**N-C**_{**6**}**H**_{**4**}**-(CH**_{**2**}**)**_{**n**}**-[Kohlenhydrat],**
**H**_{**2**}**N-CH**_{**2**}**-C**_{**6**}**H**_{**4**}**-(CH**_{**2**}**)**_{**n**}**-[Kohlenhydrat],**
**H**_{**2**}**N-C**_{**6**}**H**_{**4**}**-(CH**_{**2**}**)**_{**n**}**-O-[Kohlenhydrat],** wobei n= 1-10 ist, und
**H**_{**2**}**N-C**_{**6**}**H**_{**4**}**-(CH**_{**2**}**)**_{**m**}**-CHOH-(CH**_{**2**}**)**_{**n**}**-O-[Kohlenhydrat],** wobei m = 1-10 und n = 1-10 ist, einschliesslich der p-, o- und m-Benzen Ring Amino-lsomere, Aminomethyl-Isomere und Alkylgruppen Isomere davon;
Guanidin und Aminoguanidin Derivaten von Cellulose, Chitin, oder anderen natürlich vorkommenden nicht-absorbierbaren Kohlenhydraten bestehend aus:
**H**_{**2**}**N-C(=NH)-[Kohlenhydrat];**
**H**_{**2**}**N-C(=NH)-(CH**_{**2**}**)**_{**n**}**-[Kohlenhydrat]**, wobei n = 1-10 ist, einschliesslich der Kohlenwasserstoff Isomere, Ätherbindungs Isomere und der hydroxylierten Derivate davon;
**H**_{**2**}**N-C(=NH)-NH-[Kohlenhydrat];**
**H**_{**2**}**N-C(=NH)-NH-(CH**_{**2**}**)**_{**n**}**-[Kohlenhydrat]**, wobei n = 1-10 ist, einschliesslich der Kohlenwasserstoff Isomere und der hydroxylierten Derivate davon;
**H**_{**2**}**N-C(=NH)-NH-(CH**_{**2**}**)**_{**n**}**-O-[Kohlenhydrat],** wobei n = 1-10 ist, einschliesslich der Kohlenwasserstoff Isomere, Ätherbindungs Isomere, und der hydroxylierten Derivate davon;
**H**_{**2**}**N-C(=NH)-N=CH-(CH**_{**2**}**)**_{**n**}**-[Kohlenhydrat]**, wobei n = 1-10 ist, einschliesslich der Kohlenwasserstoff Isomere und der hydroxylierten Derivate davon;
**H**_{**2**}**N-C(=NH)-N=CH-(CH**_{**2**}**)**_{**n**}**-O-[Kohlenhydrat]**, wobei n = 1-10 ist, einschliesslich der Kohlenwasserstoff Isomere und der hydroxylierten Derivate davon;
**H**_{**2**}**N-NHC(=NH)-NH-[Kohlenhydrat];**
**H**_{**2**}**N-NHC(=NH)-NH-(CH**_{**2**}**)**_{**n**}**-[Kohlenhydrat]**, wobei n = 1-10 ist, einschliesslich der Kohlenwasserstoff Isomere und der hydroxylierten Derivate davon;
**H**_{**2**}**N-NHC(=NH)-NH-(CH**_{**2**}**)**_{**n**}**-O-[Kohlenhydrat]**, wobei n = 1-10 ist, einschliesslich der Kohlenwasserstoff Isomere, Ätherbindungs Isomere, und der hydroxylierten Derivate davon;
**H**_{**2**}**N-NHC(=NH)-N=CH-(CH**_{**2**}**)**_{**n**}**-[Kohlenhydrat]**, wobei n = 1-10 ist, einschliesslich der Kohlenwasserstoff Isomere und der hydroxylierten Derivate davon;
**H**_{**2**}**N-NHC(=NH)-N=CH-(CH**_{**2**}**)**_{**n**}**-O-[Kohlenhydrat]**, wobei n = 1-10 ist, einschliesslich der Kohlenwasserstoff Isomere, Ätherbindungs Isomere, und der hydroxylierten Derivate davon;
**H**_{**2**}**N-C(=NH)-NH-NH-[Kohlenhydrat];**
**H**_{**2**}**N-C(=NH)-NH-NH-(CH**_{**2**}**)**_{**n**}**-[Kohlenhydrat]**, wobei n = 1-10 ist, einschliesslich der Kohlenwasserstoff Isomere und der hydroxylierten Derivate davon;
**H**_{**2**}**N-C(=NH)-NH-NH-(CH**_{**2**}**)**_{**n**}**-O-[Kohlenhydrat]**, wobei n = 1-10 ist, einschliesslich der Kohlenwasserstoff Isomere, der Ätherbindungs Isomere, und der hydroxylierten Derivate davon;
**H**_{**2**}**N-C(=NH)-NH-N=CH-(CH**_{**2**}**)**_{**n**}**-[Kohlenhydrat]**, wobei n = 1-10 ist, einschliesslich der Kohlenwasserstoff Isomere und der hydroxylierten Derivate davon;
**H**_{**2**}**N-C(=NH)-NH-N=CH-(CH**_{**2**}**)**_{**n**}**-O-[Kohlenhydrat]**, wobei n = 1-10 ist, einschliesslich der Kohlenwasserstoff Isomere und der hydroxylierten Derivate davon;
d. Primäre Amine, Aminoguanidin und Guanidin Derivate von Saccharose Polyestern mit einer oder mehr funktionellen "Carbonyl-trapping" Gruppe per Molekül wobei jede funktionelle Carbonyl-trapping Gruppe in der omega-, omega-1 oder anderen isomerischen Position innerhalb der Fettsäurekette sein kann, wobei jede Fettsäurekette 3 bis 26 Kohlestoffe enthalten kann, mit ein bis fünf funktionellen Stickstoffgruppen und 1 bis 24 Hydroxylgruppen;
e. Synthetische Polysaccharide bestehend teilweise oder völlig aus Aminozuckern gebunden bei beta-1,2, beta-1,3, beta-1,4, und/oder beta-1,6 Bindungen;
f. Gemischte Polysaccharide-Polymer Derivate, worin primäre Amine, Amino-alkyl (ein bis zehn Kohlenstoffe pro Alkylgruppe), Amino-hydroxy-alkyl (ein bis zehn Kohlenstoffe pro Alkylgruppe und ein bis zehn pro Alkylgruppe), Aminoguanidin, Amino-guanidin-alkyl (ein bis zehn Kohlenstoffe pro Alkylgruppe), Amino-alkyl-guanidinyl (ein bis zehn Kohlenstoffe pro Alkylgruppe), Guanidin, Aminobenzen und/oder Aminoalkylbenzen (ein bis zehn Kohlenstoffe pro Alkylgruppe) Gruppen kovalent zu Matrizen gebunden sind, wie Epichlorohydrin Ko-Polymere von Cellulose oder Chitin und worin Kohlenwasserstoff "Spacer Gruppen" Alkene wie auch Alkylgruppen enthalten können; und
g. Nicht-Polysaccharid-Polymer Derivate, worin primäre Amine, Aminoalkyl (ein bis zehn Kohlenstoffe pro Alkylgruppe), Amino-hydroxy-alkyl (ein bis zehn Kohlenstoffe pro Alkylgruppe und ein bis zehn pro Alkylgruppe), Aminoguanidin, Amino-guanidin-alkyl (ein bis zehn Kohlenstoffe pro Alkylgruppe), Amino-alkylguanidinyl (ein bis zehn Kohlenstoffe pro Alkylgruppe), Guanidin, Aminobenzen und/oder Aminoalkylbenzen (ein bis zehn Kohlenstoffe pro Alkylgruppe) Gruppen kovalent an ein synthetisches unverdaubares Polymer gebunden sind bestehen aus Polystyrene, Styren-divinylbenzen Co-Polymer, Polyvinylalkohol und "crosslinked" Derivaten davon, und worin Kohlenwasserstoff Spacer Gruppen Alkene wie auch Alkylgruppen enthalten können; in einer mikro-fibrillten Form oder mikro-kristallinen Form mit erweiterter Oberfläche, erhöther Porösität, erhöhter Wasserspeicherungs Kapazität und erweiterter chemischer Zugänglichkeit, zur Verabreichung von besagtem Co-Agenz in einem Dosisbereich von einem Gramm/Tag bis zu 40 Gramm/Tag.

2. Die Nutzung in Bezug zu Anspruch 1, charakterisiert darin dass besagtes Co-Agenz in einer mikro-fibrillten Form oder mikro-kristallinen Form mit erweiterter Oberfläche, erhöhter Porösität, erhöhter Wasserspeicherungs Kapazität und erweiterter chemischer Zugänglichkeit ist.

3. Die Nutzung in Bezug zu Anspruch 1, charakterisiert darin, dass das hergestellte Medikament zur oralen oder intravenösen Verabreichung von besagtem Co-Agenz nützlich ist.

4. Die Nutzung in Bezug zu den Ansprüchen 1 bis 3, charakterisiert darin, dass das hergestellte Medikament zur Verabreichung des primären Agenz in einer Dosis von 10 mg/kg/Tag bis zu 1 g/kg/Tag, oder zur Verabreichung der nicht absorbierbaren Poly-Amine Substanz in einer Dosis von 10 mg/kg/Tag bis zu 1 g/kg/Tag.

## Revendications

1. Utilisation d'une composition pharmaceutique pour produire un médicament traitant les symptômes des troubles inflammatoires chroniques comprenant une pathologie basée en partie sur une augmentation de la peroxidation des lipides dans laquelle une combinaison primaire et une combinaison de co-agent produisent un effet anti-inflammatoire et analgésique, dans laquelle les troubles dont nous parlons plus haut sont sélectionnés parmi les gingivites, les periondontitis chroniques, les gastrites chroniques auto-immune, les iléites, les colites, les cystites interstitielles, l'arthrite, les tendinites, le syndrome du canal carpien et autres troubles cumulatifs des traumas, les érythematoses systémiques du lupus, les vasculites auto-immune, les asbestoses, les silicoses, les maladies pulmonaires obstructives chroniques, la maladie de Lyme, les myopathies inflammatoires, les états de mal épileptique, les neuropathies inflammatoires, les fatigues musculaires graves, ainsi que la diminution du site des oedèmes inflammatoires et le traitement des ischémies après coup et les symptomologies de réperfusion résultants d'un trauma important du système nerveux central, les attaques et les infarctus du myocarde; excluant la sclérose en plaque;
la composition est faite d'un agent de base soluble dans l'eau d'un poids moléculaire faible se situant entre 100 et 1400 et au moins d'un co-agent;
l'agent de base de la composition pharmaceutique dont nous parlons a été sélectionnée parmi:
des formules sans acide, des sels, des isomères à anneau de benzine, des dérivés d'amide, des dérivés d'ester d'acide carboxylique et des dérivés d'anneaux de benzine non-aromatique analogues 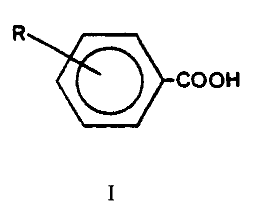
dans lequel R est sélectionné parmi
-NH₂
- groupe aminoalkyle ayant entre 1 et 10 carbones comprenant des isomères d'hydrocarbure et/ou des dérivatifs hydroxylés, comme:
-NHC(=NH)NH₂
-(CH₂)ₙ(NHC(=NH)NH₂
-(CH₂)ₙNHC(=NH)NH₂
-C(=NH)-NH₂
-(CH₂)ₙ-CH=NC(=NH)NH₂
ou n = 1-10- NHC(=NH) NHNH₂
- (CH₂)ₙ(NHC)=NH)NHNH₂
ou n = 1-10
- (CH₂)ₙ-CH=NC(=NH)NHNH₂
ou n = 1-10
- NHNHC(=NH) NH₂
- (CH₂)ₙ-NHNHC(=NH)NH₂
ou n = 1-10
- (CH₂)ₙ-CH=N-NHC(=NH)NH₂
ou n = 1-10 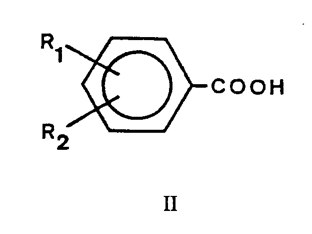 dans lequel R₁ est sélectionné parmi
- NH₂
- groupe aminoalkyle (1-10 carbones)
Y compris des isomères d'hydrocarbures et/ou des dérivés hydroxylés, comme:
- (CH₂)ₙNHC(=NH)NH₂
ou n=1-10
- C (=NH)-NH₂
- (CH₂)ₙ-CH=NC (=NH)NH₂
ou n = 1-10
-NHC(=NH)NHNH₂
- (CH₂)ₙNHC (=NH) NHNH₂
ou n = 1-10
- (CH₂)ₙ -CH=NC (=NH) NHNH₂
ou n = 1-10
- NHNHC (=NH) NH₂
- (CH₂)ₙ-NHNHC (=NH) NH₂
ou n = 1-10
- (CH₂)ₙ -CH=N-NHC (=NH) NH₂
ou n =1-10 et ou R₂ est sélectionné parmi:
- NH₂
- OH
- O-CH₃
- O-R' avec le groupe d'alkyloxy R' ayant entre 2 et 10 carbones comprenant des isomères de carbone et/ou des dérivatifs hydroxylés
- groupe aminoalkyl (1-10 carbones) comprenant des isomères de carbone et/ou des dérivatifs hydroxylés comme:
-SO₃H
-CH₃
- (CH₂)ₙCH₃ ou n = 1-10 comprenant des isomères de carbone et/ou, au lieu de cela des dérivatifs hydroxylés 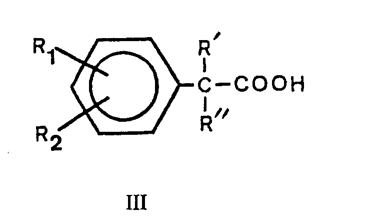 dans lequel R₁ est sélectionné parmi:
- (CH₂)ₙ-NH₂ ou n = 1-10 comprenant des isomères de carbone et/ou, des dérivatifs hydroxylés comme:
- C(=NH)-NH₂
-NHC(=NH)NH₂
- (CH₂)ₙNHC(=NH) NH₂
ou n = 1-10
ou n = 1-10
- NHC (=NH) NHNH₂
-(CH₂)ₙNHC(=NH)NHNH₂ ou n = 1-10
- (CH₂)ₙ-CH=NC(=NH)NHNH₂ ou n = 1-10
- NHNHC (=NH) NH₂
-(CH₂)ₙ-NHNHC (=NH) NH₂ ou n = 1-10
-(CH₂)ₙ-CH=N-NHC (=NH)NH₂ ou n = 1-10
dans lequel R₂ est sélectionné parmi:
- NH₂
- H
-OH
- O-CH₃
- O-R₃ ou le groupe alkyloxy R₃ a entre 2 et 10 carbones comprenant des isomères de carbone et/ou, des dérivatifs hydroxylés comme:
- Groupe aminoalkyl (1-10 carbones)- (CH₂)ₙ-CH=NC (=NH)NH₂ comprenant des isomères de carbone et/ou des dérivatifs hydroxylés comme:
-SO₃H
-CH₃
- (CH₂)ₙCH₃ ou n = 1-10 comprenant des isomères de carbone et/ou des dérivatifs hydroxylés comme
et ou R' et R" sont sélectionné parmi:
indépendamment
- H
-CH₃
-OH, Ainsi, en association avec un représentant pharmaceutique de bon standing, pour gérer l'agent de base déjà nommé d'une dose allant de 1 gramme par jour à 20 grammes par jour,
ayant pour caractéristique le fait que
le co-agent déjà nommé soit sélectionné parmi
a. des polysaccharides naturels ayant des liaisons chimiques avec beta-1,2, beta-1,3, beta-1,4 et/ou beta-1,6 contenant des sucres aminés incluant la classe chitines des biopolymères ayant la structure générale de **poly-beta-(1->4)-N-acétyle-D-glucosamine**, et porteur d'au moins un groupe d'aminés primaires mobiles;
b. des polysaccharides naturel déacetylés, ayant au moins un résidu N-acetylé, y compris du chitosan, du sulfate de chondroitine deacetylés, ou de l'acide hyalurinique deacetylés;
c. des polysaccharides aminées chimiquement sélectionnées parmi
des polysaccharides aminodesoxy tels que la cellulose 2-amino-2-desoxy; l'aminoalkyle-, amino(hydroxyalkyle)-,aminoalkyle-éther-, et amino(hydroxyalkyle-éther- dérivés de cellulose, chitine et autres hydrates de carbone naturels et non digestibles sélectionnés du groupe suivant:
H₂N-(CH₂)ₙ-[hydrate de carbone] ou n = 1-10, incluant les isomères d'alkyle
H₂N-(CH₂)ₘ-CHOH-(CH₂)N-[hydrate de carbone] ou m = 0-10 et n = 0-10
H₂N-(CH₂)ₙ-O-[hydrate de carbone] ou n = 1-10
H₂N-(CH₂)ₘ-CHOH-(CH₂)n-O-[hydrate de carbone] ou m = 0-10 et n = 0-10
des aminobenzyles-dérivés de cellulose, chitine ou autres hydrates de carbone naturels et non digestibles sélectionnés dans le groupe suivant:
H₂N-C₆H₄-(CH₂)ₙ-[hydrate de carbone)
H₂N-CH₂-C₆H₄-(CH₂)ₙ-[hydrate de carbone)
H₂N-C₆H₄-(CH₂)ₙ-O-[hydrate de carbone) ou n = 0-10 et
H₂N-C₆H₄-(CH₂)ₘ-CHOH-(CH₂)ₙ-O- [hydrate de carbone) ou m = 0-10
et n = 0-10, comprenant des anneaux d'amino-isomères p-, 0- ou des isomères du groupe alkyle;
des aminobenzyles-dérivés de cellulose, chitine ou autres hydrates de carbone naturels et non digestibles sélectionnés dans le groupe suivant:
H₂N-C₆H₄-(CH₂)ₙ-[hydrate de carbone)
H₂N-CH₂-C₆H₄-(CH₂)ₙ-[hydrate de carbone)
H₂N-C₆H₄-(CH₂)ₙ-O-[hydrate de carbone) ou n = 0-10 et
H₂N-C₆H₄-(CH₂)ₘ-CHOH-(CH₂)ₙ-O- [hydrate de carbone) ou m = 0-10
et n = 0-10, comprenant des anneaux d'amino-isomères p-, 0- ou des isomères du groupe alkyle;
des dérivés de cellulose guanidine et aminoguanidine,
de la chitine ou d'autres hydrates de carbone non-absorbable et naturels que l'on choisira parmi:
H₂N-C(=NH)-[hydrate de carbone]
H₂N-C(=NH)-O-(CH₂)ₙ-[hydrate de carbone], où n = 1 -10, comprenant des isomères d'hydrate de carbone et des dérivés hydroxyles
H₂N-C(=NH) -O- ( CH₂)ₙ-[hydrate de carbone], où n = 1 -10 comprenant des isomères d'hydrate de carbone, des isomères lies à l'éther et des dérivés hydroxyles;
H₂N-C( =NH )-NH-[hydrate de carbone];
H₂N-C( =NH)-NH- (CH₂)ₙ₋[hydrate de carbone], où n = 1 -10, comprenant des isomères d'hydrate de carbone et des dérivés hydroxyles
H₂N-C( =NH)-NH- (CH₂)ₙ₋0₋[hydrate de carbone], où n = 1-10, comprenant des isomères d'hydrate de carbone, des isomères lies à l'éther et des dérivés hydroxyles;
H₂N-C(=NH)-N=CH-(CH₂)ₙ-[hydrate de carbone], où n = 10, comprenant des isomères d'hydrate de carbone et des dérivés hydroxyles
H₂N-C(=NH)-N=CH-(CH₂)ₙ-O-[hydrate de carbone], où n = 1-10, comprenant des isomères d'hydrate de carbone et des dérivés hydroxylés;
H₂N-NHC(=NH)-NH-[hydrate de carbone];
H₂N-NHC(=NH)-NH-(CH₂)ₙ-[hydrate de carbone], où n = 1-10, comprenant des isomères d'hydrate de carbone et des dérivés hydroxylés;
H₂N-NHC(=NH)-NH=(CH₂)ₙ-O-[hydrate de carbone], où n = 1-10, comprenant des isomères d'hydrocarbure, des isomères lies à l'éther et des dérivés hydroxylés;
H₂N-NHC(=NH)-N=CH-(CH₂)ₙ₋[hydrate de carbone], où n = 1-10, comprenant des isomères d'hydrocarbure et des dérivés hydroxylés;
H₂N-NHC(=NH)N=CH-(CH₂)ₙ-O-[hydrate de carbone], où n =1-10 comprenant des isomères d'hydrocarbure, des isomères liés à l'éther et des dérivés hydroxylés
H₂N-C(=NH)-NH-NH-[hydrate de carbone]
H₂N-C(=NH)-NH-NH-(CH₂)ₙ₋[hydrate de carbone], où n = 1-10, comprenant des isomères d'hydrocarbure et des dérivés hydroxylés;
H₂N-C(=NH)-NH-NH-(CH₂)ₙ-O-[hydrate de carbone], où n = 1-10 comprenant des isomères d'hydrocarbure, des isomères liés à l'éther et des dérivés hydroxylés
H₂N-C(=NH)-NH-N-CH-(CH₂)n-[hydrate de carbone], où n = 1-10, comprenant des isomères d'hydrocarbure et des dérivés hydroxylés;
H₂N-C(=NH)-NH-N=CH-(CH₂)ₙ-O-[hydrate de carbone], où n = 1-10 comprenant des isomères d'hydrocarbure, des isomères liés à l'éther et des dérivés hydroxylés
d. L'amine primaire, l'aminoguanidine et la guanidine dérivés des polyesters de saccharose ayant un groupe fonctionnel piégeant un ou plusieurs carbonyles par molécule dans lequel chaque groupe fonctionnel piégeant le carbonyle est dans la position isomérique oméga-, oméga-1 ou autre, à l'intérieur des chaînes d'acyle gras, dans laquelle chaque chaîne d'acyle gras peut avoir entre 3 et 26 carbones, entre 1 et 5 groupes fonctionnels de nitrogène et entre 1 et 24 groupes d'hydroxyle;
g. Des dérivés polymériques de non-polysaccharide dans lesquels une amine primaire, de l'aminoalkyle (un à 10 carbones par groupe d'alkyle), de l'amino hydroxyalkyle(1 à 10 carbones par groupe d'alkyle et 1 à 10 groupes d'hydroxyle par groupe d'alkyle, de l'aminoguanidine, de l'aminoguanidinylalkyle (1 à 10 carbones par groupe d'alkyle), de l'aminoaikyie-guanidinyle (1 à 10 carbones par groupe d'alkyle), de la guanidine, de l'aminobenzène et/ou de l'aminoalkylbenzène (1 à 10 carbones par groupe d'alkyle) sont attachés de façon covalente à un polymère synthétique non digestible sélectionné parmi : le polystyrène, le copolymère de styrène-divinylbenzène, l'alcool de polyvinyle et des dérivés à liaison croisée, et dans lesquelles des groupes barres d'hydrocarbure peuvent comprendre de l'alcène ainsi que des groupes d'alkyle; sous forme microfibrillée ou sous forme microcrystalline ayant fait ressortir la surface, augmenté la porosité, augmenté la capacité à retenir l'eau et fait ressortir l'accessibilité chimique à l'administration du dit co-agent pour une dose entre 1 à 40 grammes par jour.

2. L'utilisation selon la demande 1, se **caractérise par le fait que** le dit co-agent se trouve sous une forme microfibrillée ou microcrystalline ayant fait ressortir la surface, augmenté la porosité, augmenté la capacité à retenir l'eau et ayant fait ressortir l'accessibilité chimique.

3. L'utilisation de la demande 1 se **caractérise par le fait que** le médicament produit est utilisable par voie orale ou intraveineuse du dit co-agent.

4. L'utilisation selon les demandes 1 à 3, se **caractérise par le fait que** le médicament produit est utilisable pour l'administration de l'agent de base dans une dose de 10 mg/kg/jour à 1.0 g/kg/jour, ou pour l'administration de la substance polyamine qui ne peut pas être digérée dans une dose de 10 mg/kg/jour à 1.0g/kg/jour.
